(19) Europäisches Patentamt
European Patent Office
Office européen des brevets

(11) **EP 4 663 113 A1**

(12) **EUROPEAN PATENT APPLICATION**

(43) Date of publication:
**17.12.2025 Bulletin 2025/51**

(21) Application number: **24181791.5**

(22) Date of filing: **12.06.2024**

(51) International Patent Classification (IPC):
**A61B 5/022** (2006.01)        **A61B 5/023** (2006.01)

(52) Cooperative Patent Classification (CPC):
**A61B 5/022; A61B 5/02141; A61B 5/02233; A61B 5/0225; A61B 5/023;** A61B 2562/0247; A61B 2562/168

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **PFEIFFER, Ulrich**
  **5656 AG Eindhoven (NL)**
• **GERG, Maximilian Werner**
  **5656 AG Eindhoven (NL)**
• **HOFFMANN, Benjamin**
  **5656 AG Eindhoven (NL)**
• **WIMMER, Julia**
  **5656 AG Eindhoven (NL)**
• **FAEHLE, Matthias**
  **5656 AG Eindhoven (NL)**

(74) Representative: **Philips Intellectual Property & Standards**
**High Tech Campus 52**
**5656 AG Eindhoven (NL)**

(54) **PRESSURE MEASURING DEVICE TO BE USED FOR DETERMINING A PHYSIOLOGICAL PARAMETER OF A SUBJECT**

(57)    The invention relates to a pressure measuring device configured to surround a subject's body part. It comprises a pressure application element configured to apply pressure to the body part, a pressure sensing device configured to measure a pressure signal of the body part and a shell 4 which is arranged to be located between the pressure application element and the body part. The inner surface of the shell comprises a pressure sensing area 72 in which at least a part of the pressure sensing device is located. Preferentially, the pressure sensing area 72 is closer to the distal edge 124 of the shell than to the proximal edge 125 of the shell. Placing the pressure sensing area in this way can allow for an improved pressure signal.

Fig. 12

EP 4 663 113 A1

**Description**

FIELD OF THE INVENTION

The invention relates to a pressure measuring device configured to surround a subject's body part, a pressure measuring method for measuring pressure of the subject by using the pressure measuring device, and an apparatus, method and computer program for determining a physiological parameter like the subject's blood pressure.

BACKGROUND OF THE INVENTION

WO 2014/121945 A1 discloses a blood pressure measuring system configured to surround a subject's body part, wherein the blood pressure measuring system comprises pressurization means for applying pressure to the body part and a kinking-proof shell that is arranged so as to be located between the pressurization means and the body part. The blood pressure measuring system further comprises a pressure sensing device configured to measure a tissue pressure signal of the subject, which is indicative of blood pulsations, wherein this measured tissue pressure signal is used for determining the blood pressure.

SUMMARY OF THE INVENTION

It is an object of the present invention to provide a pressure measuring device which allows for an improved measurement of a pressure signal of a subject. It also is an object of the present invention to provide a corresponding pressure measuring method for measuring pressure of the subject by using the pressure measuring device, and an apparatus, method and computer program for determining a physiological parameter like the subject's blood pressure based on the measured pressure.

In a first aspect of the invention a pressure measuring device configured to surround a subject's body part is presented, wherein the pressure measuring device comprises a) a pressure application element configured to apply pressure to the body part, b) a pressure sensing device configured to measure a pressure signal of the subject, wherein the measured pressure signal is indicative of blood pulsations, c) a shell arranged to be located between the pressure application element and the body part, when the pressure measuring device surrounds the body part, wherein the shell is curled to a cylindrical or conical configuration, wherein the inner surface of the shell comprises a pressure sensing area in which at least a part of the pressure sensing device is located. The shell comprises a distal edge and a proximal edge, wherein preferentially the pressure sensing area is closer to the distal edge of the shell than to the proximal edge of the shell.

Often an occlusion of an artery happens more distally, especially if the shell is curled to a conical configuration, wherein for this reason locating the pressure sensing area closer to the distal edge of the shell than to the proximal edge of the shell can lead to an improved pressure signal.

Moreover, preferentially, also the pressure sensing area comprises a distal edge and a proximal edge, wherein the distance between the distal edge of the pressure sensing area and the distal edge of the shell is within a range from 15% to 20%, more preferred it is 17% and even further preferred 17.2%, of the entire height of the shell from its distal edge to its proximal edge and/or the distance between the distal edge of the pressure sensing area and the distal edge of the shell is within a range from 20 mm to 30 mm, even more preferred it is 25 mm. Especially in this case, in an example, the pressure sensing area has a length between 65 mm and 75 mm, preferentially a length of 70 mm, in the circumferential direction and/or a length between 35 mm and 45 mm, preferentially a length of 40 mm, in the proximal-distal direction. In relative terms, especially if the distances between the edges of the pressure sensing area and the shell are as described at the beginning of this paragraph, the pressure sensing area preferentially has a length between 20% and 50% and most preferably between 24% and 26% of the inner circumference of the shell in the circumferential direction and a length between 15% to 40%, preferentially a length of 27.5%, of the entire shell height in the proximal-distal direction. In this case the inner circumference of the shell refers to a situation when no force is applied to the shell, i.e., when the pressure measuring device lies "free" on a table. It has been found that these features allow for a further improved acquisition of the pressure signal from the surrounded body part.

The pressure sensing area preferentially is rectangular, wherein the pressure sensing area preferentially is three-dimensionally curved due to its location on the inner surface of the shell that has been curled to be in a cylindrical or conical configuration. Two parallel side edges of the pressure sensing area are preferentially parallel to the distal and proximal edges of the shell. The other parallel side edges, which are perpendicular to the first parallel side edges, are preferentially parallel to the imaginary longitudinal axis of the cylindrical or conical configuration of the curled shell.

The pressure sensing device can comprise a fluid-filled pressure sensing pad arranged in the pressure sensing area on the inner surface of the shell. In particular, the pressure sensing pad can be filled with fluid having a fluid thickness, in a flat condition of the pressure sensing pad, within a range from 0.90 mm to 1.10 mm. In a preferred embodiment, this thickness is 1.07 mm. It has been found that these features allow for a further optimized measurement performance. In particular, they can prevent a pressure offset when surrounding the body part, even if the circumference of the body part is relative small, minimize wrinkles and the likelihood of separation of the fluid within the pressure sensing pad into different disconnected sub-amounts of the fluid. The fluid thickness is the thickness of the fluid only, i.e., without considering the thickness of layers of the pressure sensing pad. The fluid preferentially is a liquid. However, the fluid can also be a gas.

In an example a mesh is arranged within the pressure sensing pad, wherein preferentially the mesh is a three-dimensional mesh. This allows to prevent, for instance, a closure of a fluid tube at a connection to the pressure sensing pad. It also allows to prevent the formation of non-fluid-filled compartments within the pressure sensing pad. This allows for a further improved measurement of the pressure signal, which finally can lead to a further improved determination of the physiological parameter based on the improved pressure signal.

Preferably, the pressure sensing device comprises a pressure transducer located in a pressure transducer housing and a fluid-filled tube for guiding pressure from the pressure sensing pad to the pressure transducer. It is preferred that a) the pressure sensing pad and the tube and b) the pressure transducer housing are filled with different fluids having different viscosities. In particular, the fluid in the pressure sensing pad and the tube can have a lower viscosity than the fluid in the pressure transducer housing. For instance, a) the pressure sensing pad and the tube are filled with silicon oil and/or b) the pressure transducer housing is filled with glycerol. The low viscosity fluid in the pressure sensing pad and the tube can lead to a reduced dry out and allow for a good pressure transmission to the pressure transducer housing. The high viscosity fluid in the pressure transducer housing generally works well together with the pressure transducer, wherein the pressure from the low viscosity fluid can be effectively transmitted to the high viscosity fluid via the interface formed between these two fluids. These features therefore also allow for an improved measurement of the pressure signal, i.e., they allow for a high-accuracy pressure sensing device, and allow for an increased lifetime of the high-accuracy pressure sensing device as well.

In an example the pressure measuring device comprises a mark having an angular offset within a range from 35° to 45 °, preferentially of 40°, relative to the location of the center of the pressure sensing area. Preferably the angular offset is with respect to an imaginary rotational axis coinciding with the axis of the cylindrical or conical configuration of the curled shell. If a medical person like a physician arranges the pressure measuring device around the body part such that the mark is located above the artery, particularly the brachial artery, the pressure sensing area is located such relative to the body part that the acquisition of the pressure signal from the body part can be further improved and also tolerates slight misalignments best.

In an embodiment, the pressure measuring device comprises two of these marks with an angular offset. In particular, the pressure measuring device can comprise a first mark having an angular offset within a range from 35° to 45°, preferentially of 40°, relative to the location of the center of the pressure sensing area, and the pressure measuring device can comprise a second mark having an angular offset within a range from 35° to 45°, preferentially of 40°, relative to the location of the center of the pressure sensing area, wherein the angular offset of the first and second marks is in opposite angular directions. For instance, the angular offset of the first mark can be clockwise and the angular offset of the second mark can be counter-clockwise or vice versa. For instance, if the body part is an upper arm and when arranging the pressure measuring device around the upper arm, the pressure measuring device can be arranged such that the first mark coincides with the brachial artery, if the upper arm is the left upper arm, and such that the second mark coincides with the brachial artery, if the upper arm is the right upper arm. As mentioned above, using the arterial marks for positioning the pressure measuring device improves the quality of the measured pressure signal and tolerates slight misalignments best.

Preferably, an elastic liner is arranged inside of the curled shell when the pressure measuring device surrounds the body part, wherein, if the pressure measuring device does not surround the body part, there is free space in between the liner and the inner surface of the shell. Due to its elasticity in combination with the free space between the liner and the inner surface of the curled shell, the likelihood of wrinkles of the liner and thus of pressure marks caused by wrinkles can be reduced. The elasticity can also avoid tension on the surrounded body part that might lead to venous congestion, wherein preferentially nevertheless the liner surrounds the body part tight enough to avoid wrinkles also for a smallest allowed circumference of the body part for which the pressure measuring device might be specified.

The liner can also ensure that any direct contact between the shell and the body part is prevented. In particular, the liner may ensure that any friction between the shell and the body part with the intermediate liner is relatively small. Also, the liner may ensure that any relative motion of the shell with respect to the body part does not evoke squeezing or pinching of the body part.

Preferably, if the pressure measuring device does not surround the body part, the free space in between the liner and the inner surface of the shell has a maximum distance of at least 0.5 cm and/or has a maximum distance being smaller than or equal to 2.0 cm. In an embodiment, this maximum distance is 1.0 cm. Moreover, preferably, if the pressure measuring device does not surround the body part, the liner is in contact with the inner surface of the shell only at the distal and proximal edges of the shell or not in contact at all with the inner surface of the shell. It is also preferred that, if the pressure measuring device does not surround the body part, the maximum distance between the liner and the inner surface of the shell is in a central region in between the proximal and distal edges of the shell. Moreover, if the pressure measuring device does not surround the body part, preferentially the distance decreases from the center to the proximal edge and to the distal edge of the shell non-linearly and/or monotonically. In an example, if the pressure measuring device does not surround the body part, the liner forms a convex curve in an imaginary plane that completely comprises an imaginary central axis of the shell, around which the shell is curled. It has been found that these features can allow for a further reduction of the likelihood of generating pressure marks.

Preferentially, the liner comprises or is made of antimicrobial material. This reduces the likelihood of infections.

Preferentially, the liner is a textile or textile-like layer which is arranged so as to be located between the shell and the body part. The liner could also be regarded as being a fabric.

The textile or textile-like layer can be a multifilament textile or textile-like layer. The textile or textile-like layer may be arrased or machine-knit. By using an arrased or machine-knit textile or textile-like layer, the friction between the body part and the shell with the intermediate liner may be further reduced.

It is also preferred that the liner is made of polyamide or of a mix of elastane and polyester. For instance, the liner can be a textile or textile-like layer of polyamide, especially a polyamide velour, or the liner can be a textile or textile-like layer of a mixture of elastane and polyester. It is also possible that the liner is made of a material comprising a percentage of nylon, 70D, within the range from 80% to 90%, and a percentage of nylon Spandex, 40D, within a range from 10% to 20%. In a preferred embodiment the material comprises 86% nylon, 70D, and 14% nylon Spandex, 40D. Moreover, in an example the liner is made of a material that has been produced by knitting. In particular, the material can be of the weft knit type. The weft knit type preferentially refers to two yards in single knit jersey structure and similar. It has been found that these materials allow for a good avoidance of pressure marks on the skin of the surrounded body part and also allow for a good transmission of the pressure signal through the liner material to the pressure sensing area. Thus, a high quality pressure measurement can be carried out, without causing pressure marks on the skin of the surrounded body part.

The liner is arranged between the skin of the body part and the shell preferentially for preventing pinching of the skin and of hairs. However, besides causing pressure marks due to wrinkles, generally a liner between the skin and the pressure sensing part of the pressure sensing device on the inner surface of the shell could dampen the tissue pressure waves and cause signal loss, as indicated before. For this reason, the liner can have yarn threads that are strong, but flexible, and a structure, i.e., for instance, a knitting, which allows for a relatively small thickness of the liner and which reduces dampening air inclusions when applied to the body part. For these reasons the above described materials are preferred and preferentially, if the liner comprises yarn threads, they preferentially have a thickness within the range from 0.10 to 0.25 mm, further preferred within the range from 0.13 to 0.23 mm, wherein preferentially the liner has been produced by knitting.

The thickness of the liner 93 preferentially is within the range from 0.3 to 0.5 mm. This allows for a further improved transmission of the pressure signal from the tissue to the pressure sensing area.

The liner, which preferentially is a textile or textile-like layer, may be provided with high stretchability. In an embodiment, the material of the liner has a stretchability of at least 50%, more preferred of at least 80% and even further preferred of 100%. This stretchability refers to at least one direction of the liner and preferentially to all directions of the liner. It has been found that this stretchability allows for a further improved avoidance of the wrinkles and hence pressure marks on the skin of the surrounded body part.

The liner can be sock-like, hose-like or stocking-like. It can cover one or two surfaces, i.e., the inner and outer surface, of the shell. The liner can be partly or completely flocked, i.e., the liner can be flocked at least in sections. Flocking might make the blood pressure measurement more comfortable for the subject.

It is preferred that the pressure application element includes a fluid bag, wherein the fluid bag is fillable with fluid for applying the pressure to the body part, wherein the fluid bag comprises compartments which are separated by compartment walls with an opening such that the fluid can flow from compartment to compartment.

Since the fluid bag comprises compartments which are separated by compartment walls with an opening such that the fluid can flow from compartment to compartment, a more uniform application of the pressure on the surrounded body part is possible. This also can lead to a more accurate relation between the applied pressure and the measured pressure signal, wherein this more accurate relation finally can lead to an improved determination of a physiological parameter of the subject like the blood pressure.

The fluid, with which the fluid bag is filled, can be a gas like air or a liquid.

Moreover, the compartment walls preferentially are perpendicular to the proximal and distal edges of the fluid bag, and preferentially the respective opening in the respective compartment wall is arranged in the center of the respective compartment wall. In particular, the respective opening in the respective compartment wall can have a height within a range from 25% and 50% of the height of the fluid bag, wherein the height of the fluid bag is measured as the distance between the proximal and distal edges of the fluid bag. It has been found that these features allow for an even more uniform application of the pressure on the surrounded body part, which can finally lead to a further improved determination of a physiological parameter of the subject like the blood pressure

Furthermore, preferentially the fluid bag has an end portion in which no fluid flowable. Preferentially, the end portion has a length in the lateral direction within a range from 20% to 25% of the entire lateral length of the fluid bag, when the fluid bag is in a flat, unrolled and non-filled condition. The end portion, in which no fluid is flowable, can have a fastening means like a hook-and-loop fastener element for fastening this end portion to the opposing other lateral end portion which preferentially also comprises a corresponding fastening element. The fastening can be used, after the shell has been wrapped around the subject's body part. Using a part of the fluid bag for fastening purposes allows to reducing the efforts for manufacturing the fluid bag, because no separate additional fastening component is necessarily needed. The length of the end portion within a range from 20% to 25% of the entire lateral length of the fluid bag, when the fluid bag is in a flat, unrolled and non-filled condition, allows for a very good pressure application to the body part and at the same time for a reliably fastening of the pressure measuring device.

The fluid bag can have a shape that follows a curve element, when the fluid bag is in a flat, unrolled and non-filled condition. The lateral direction then follows this curve element. This shape of the fluid bag can allow for an even more uniform application of the pressure on the surrounded body part, especially in a conical configuration in which the shell is conically.

Preferentially, within the fluid bag one or several opening elements are arranged, which are configured to keep a respective opening in a respective compartment wall open. The one or several opening elements are arranged at least at the locations of the openings. In an example a single opening element is arranged within the fluid bag and keeps the openings open. The one or several opening elements can be regarded as being one or several inlays. This can allow for an even more uniform application of the pressure on the surrounded body part.

The one or several opening elements are preferentially arranged such within the fluid bag that they do not contact the compartment walls. Moreover, preferentially the one or several opening elements are arranged centrally within the respective opening. It has been found that also this allows for an even more uniform application of the pressure on the surrounded body part.

Furthermore, preferentially the one or several opening elements are arranged such within the fluid bag that they are not movable relative to the compartment walls. For instance, the one or several opening elements can be stuck and/or adhered to one or two inner surfaces of the fluid bag. This allows to better ensure that the one or several opening elements are optimally placed within the fluid bag.

The one or several opening elements preferentially include a textile material. Hence, the material of the one or several opening elements can be a textile material. The textile material can be polyamide textile material. For example, the material of the one or several opening elements can be a polyamide velour material. Thus, in an embodiment an inlay made of a polyamide velour material is arranged within the fluid bag with a distance to the compartment walls, in order to keep the respective opening open, wherein preferentially the inlay is stuck to an inner surface of fluid bag. It has been found that by using a textile material the pressure can be even more uniformly applied on the surrounded body part.

Preferentially, the one or several opening elements have length within a range from 65% to 95% and further preferred within a range from 70% to 95% of the entire length of the part of the fluid bag, in which the fluid can flow, in the lateral direction, if the fluid bag is unwrapped and flat. In an embodiment the length of the one or several opening elements is 70% or 77% of the entire length of the part of the fluid bag, in which the fluid is flowable, in its flat unwrapped condition and in the lateral direction. In particular, if as an opening element an inlay is used, preferentially the inlay has a length within a range from 65% to 95% and further preferred within a range from 70% to 95% of the entire length of the part of the fluid bag, in which the fluid can flow, in the lateral direction, if the fluid bag is unwrapped and flat. In an embodiment the length of the inlay is 70% or 77% of the entire length of the part of the fluid bag, in which the fluid is flowable, in its flat unwrapped condition and in the lateral direction. As mentioned above, the lateral direction follows the curvature of the fluid bag, if the fluid bag is curved in its unwrapped flat condition. This curvature of the unwrapped fluid bag might the present, if the wrapped shell is conical. It has been found that this also allows for a further improved distribution of the fluid within the fluid bag.

The fluid bag can comprise two foils, which are welded to each other at the edges of the fluid bag and also at the locations of the compartment walls, i.e., the compartment walls can be formed by the welding of the two foils together at the locations at which the compartment walls should be present. In other words, they can be formed by the welding joints. This allows to manufacture the fluid bag easier, because no additional elements for forming the compartment walls are required.

The shell is curled to a conical or cylindrical configuration, wherein the shell can comprise pull tabs which are arranged such that, if a user pulls the pull tabs, the diameter of the curled shell is extended. Since the shell can comprise the pull tabs which are arranged such that, if a user pulls the pull tabs, the diameter of the shell is extended, the diameter of the pressure measuring device can be easier adapted to different widths or thicknesses of surrounded body parts of different subjects.

In an example, the shell comprises two circumferential end portions, wherein one of the pull tabs is attached to one of the circumferential end portions of the shell and another one of the pull tabs is attached to the outer surface of the shell in between the two circumferential end portions of the shell. It has been found that this allows for an even easier adaptation of the diameter to be carried out by medical personnel.

Preferentially, the pull tabs are elongated and have a longitudinal axis being perpendicular to the proximal-distal direction. This shape of the pull tabs leads to an application of forces, when the medical personnel pulls the pull tabs, which further facilitates the adaptation of the diameter.

In an example, the pull tabs are integrally formed together with attachment elements, wherein the attachment elements are attached to the shell. In particular, the respective attachment element has a rectangular shape, wherein the respective pull tab is adjacent to one side of the rectangular shape being parallel to a proximal-distal direction. In an example, the corners of the respective attachment element towards the respective pull tab are rounded. The attachment elements can be directly attached to the outer surface of the shell and/or the attachment elements can be attached to the outer surface of the shell via a further attachment element that is attached to the attachment elements and the outer surface of the shell. This further attachment element can be an attachment layer like a gluing foil or a gluing fabric that covers and thereby attaches to the outer surface of the shell and also to the attachment elements. In a preferred embodiment, the attachment layer covers and thereby attaches to the entire region of the outer surface of the shell in between the attachment elements. Moreover, the attachment layer can extend from the proximal edge to the distal edge of the shell. These features allow for a reliable and long-lasting attachment of the pull tabs to the shell, wherein the pressure measuring device can still be manufactured with acceptable efforts.

The two circumferential end portions preferentially are overlapping. Moreover, preferentially at least one of the circumferential end portions is tapered such that the dimension of the at least one of the circumferential end portions in the height direction, which is the direction of the curling axis, decreases towards the outer end edge of the at least one of the circumferential end portions. Since at least one of the circumferential end portions can be tapered, the two circumferential end portions can be better overlapped such that one of the circumferential end portions does not protrude relative to the other of the circumferential end portions in the proximal-distal direction which corresponds to the axial direction and the height direction. Thus, such a protrusion, which might engage with other elements like a possible elastic liner and thereby hinder sliding of the two circumferential end portions on each other, can be avoided. Especially this hindrance would be disadvantageous particularly in the case of inflation.

Preferentially, the tapering of the at least one of the circumferential end portions starts between the outer edge of the at least one of the circumferential end portions and the location of the pressure sensing area. Since the tapering starts between the outer lateral edge of the at least one of the lateral end portions and the location of the pressure sensing area, the tapering does not influence the distance between the pressure sensing area and the edges of the shell, which are substantially parallel to the proximal-distal direction. This constant distance can contribute to a high quality of the pressure signal.

Preferentially, at least a part of at least one of proximal and distal edges of the tapered at least one of the circumferential end portions follows a spline function and/or a function forming an S curve. It also preferred that the tapering of the at least one of the circumferential end portions of the shell is realized by defining at least four regions, a) a first region at the location of the pressure sensing area at which the height of the shell has a constant first value, b) a second region being a transition region in which the height changes from the first value to a second value being smaller than the first value, c) a third region in which the height has the constant second value and d) a fourth region in which the height decreases for forming rounded corners at the outer lateral edge of the shell. In an example, the change of the height in the second region follows a spline function or a function forming an S curve. It has been found that by using such a type of tapering, the two circumferential end portions can be even better overlapped such that one of the circumferential end portions does not protrude relative to the other of the circumferential end portions in the proximal-distal direction.

It is preferred that the third and fourth regions do not comprise proximal and distal end portions being bent away from the inside of the curled shell. It is further preferred that also the second region does not comprise proximal and distal end portions being bent away from the inside of the curled shell. Generally, it is preferred that in the region of the at least one of the circumferential end portions, which is tapered, a proximal end portion and/or a distal end portion is not bent away from the inside of the curled shell. This allows for a further improved movement of the circumferential end portions relative to each other and hence to a further improved modification of the circumference of the shell and hence of the pressure measuring device.

In an example, at least one of the proximal and distal end portions of the shell is at least partly bent away from the inside of the curled shell and/or at least one of the proximal and distal end portions is softer than a main part of the shell located between the proximal and distal end portions. For instance, the above mentioned first region may comprise a proximal end portion and/or a distal end portion being bent away from the inside of the curled shell and/or being softer than the main part. Since, in an example, at least one of the proximal and distal end portions is at least partly bent away from the body part, when the pressure measuring device surrounds the body part, the likelihood of pressure marks on the body part can be reduced. Also that at least one of the proximal and distal end portions can be softer than a main part of the shell, wherein the main part of the shells is located between the proximal and distal end portions, can lead to a reduced likelihood of pressure marks on the body part.

If in an example at least one of the proximal and distal end portions of the shell is at least partly bent away from the inside of the curled shell, the attachment layer may extend from the proximal end portion to the distal end portion, without covering a bending of the at least one of the proximal and distal end portions. Thus, in this case the attachment layer may not reach from the proximal edge to the distal edge, but end immediately before the respective bent region starts. This avoids that the attachment layer needs to be attached, for instance, glued, to a curve surface, which can reduce manufacturing efforts and also increase the lifetime of the attachment and hence of the pressure measuring device.

The shell preferentially is a kinking-proof shell. The shell can be curled to a cylindrical or conical configuration, as also mentioned above. The curled cylindrical or conical shell surrounds and encases the body part, when the blood pressure should be measured. Moreover, preferentially, the pressure sensing device is configured to measure the pressure signal on the skin of the encased part of the subject, i.e., on the skin of the surrounded body part, while the pressure application element increases or decreases the applied pressure.

In an example the height of the bent distal end portion is within a range from 6.3 mm to 7.3 mm, preferentially within a range from 6.37 mm to 7.25 mm, and the width of the bent distal end portion is within a range from 3.2 mm to 5.0 mm, wherein the height is measured in the proximal-distal direction and the width is measured in a radial direction. Moreover, in an example the height of the bent proximal end portion is within a range from 3.9 mm to 4.5 mm, especially within a range from 3.96 mm to 4.47 mm, and the width of the bent proximal end portion is within a range from 2.3 mm to 4.0 mm. It has been found that these dimensions allow for a further reduction of the likelihood of pressure marks on the surrounded body part especially of an adult with a medial circumference of the body part within the range from 26 cm to 34 cm. Preferentially, for a body part having a medical circumference being outside of this range, the height and width ranges mentioned above include smaller or larger values.

In an example the height of the bent distal end portion is within a range from 3% to 8%, further preferred from 4% to 7% and even further preferred from 5% to 6% of the total shell height, and/or the width of the bent distal end portion is within a range from 4% to 9%, further preferred from 5% to 8% and even further preferred from 6.5% to 7.5% of the distal circumference of the shell, wherein the height is measured in a proximal-distal direction and the width is measured in a radial direction. Moreover, in an example the height of the bent proximal end portion is within a range from 1% to 6%, further preferred from 2% to 5% and even further preferred from 3% to 4% of the total shell height, and/or the width of the bent proximal end portion is within a range from 1% to 6%, further preferred from 2% to 5% and even further preferred from 3.5% to 4.5% of the proximal circumference of the shell. It has been found that these dimensions allow for a further reduction of the likelihood of pressure marks on the surrounded body part.

The distal circumference of the shell is measured at the distal end immediately before the distal end starts to bend away from the body part if such a bend is present. The proximal circumference is measured at the proximal end of the shell immediately before the proximal end portion is bent away from the body part if such a bend is present. If such a bend is not present, the circumferences refer to the distal and proximal edges, respectively, of the shell. Moreover, the circumferences refer to the situation in which the pressure measuring device is in the cylindrical or conical configuration, without being wrapped around the body part and without applying any pressure to the shell. Thus, the circumferences refer to a "free" state of the pressure measuring device with the curled shell.

The circumferential end portions can also be regarded as being lateral end portions referring to a lateral direction, i.e., they can be regarded as being end portions in the lateral direction that is perpendicular to the proximal-distal direction, if it were assumed that the shell would be uncurled and pressed flat on a table. Similarly, they can be regarded as being the end portions in the circumferential direction.

In an example, as mentioned above, the tapering of the lateral end portion of the shell is realized by defining at least four regions, i.e., a) a first region at the location of the pressure sensing area at which the height of the shell has a constant first value, b) a second region being a transition region in which the height changes from the first value to a second value being smaller than the first value, c) a third region in which the height has the constant second value and d) a fourth region in which the height decreases for forming rounded corners at the outer lateral edge of the shell. As also mentioned above, the change of the height in the second region preferentially follows a spline function or a function forming an S curve, i.e., a function having an inflection point. Moreover, preferentially the third and fourth regions do not comprise proximal and distal end portions being bent away from the body part. Furthermore, at least in the fourth region the thickness, i.e., the material thickness, of the shell preferentially is smaller than in the first region. In the fourth region the thickness can be decreasing towards its lateral outer edge.

The pressure measuring device preferentially is configured such that, when pressure is applied to the body part by the pressure application element, the overlapping portions of the shell, i.e., the overlapping lateral or circumferential end portions of the shell, can move or slide relative to each other, thereby reducing the diameter of the shell. This moving or sliding can be further improved by the optional reduced material thickness of at least one of the lateral or circumferential ends of the shell.

In an example the shell comprises an opening, in which the tube is arranged, and a tube housing on the outside surface of the shell adjacent to the location of the opening for housing a part of the tube. Preferentially, the tube housing protrudes from the outside of the shell. Moreover, the tube housing can have a lengthy portion being formed like a part of a hollow cylinder and an adjacent tapered portion. The tube housing can have an igloo-like shape. These features reduce the likelihood of a kink in the tube and they also can back a fluid filled active area of the pressure sensing pad, thereby allowing for a further improved measurement of the pressure from the body part. Moreover, these features also allow to reduce the likelihood of pressure marks on the surrounded body part, which generally might be caused, inter alia, by a part of the tube being on the inner surface of the shell.

In an example the pressure sensing device comprises a pressure transducer integrated in the shell or located between the shell and the pressure sensing pad, wherein the pressure transducer is located below the pressure sensing pad. In particular, a pressure transmitting material, especially a fluid, can be arranged between the pressure sensing pad and the pressure transducer for transmitting pressure from the pressure sensing pad to the pressure transducer. It is also possible that the pressure transducer is in direct contact with the pressure sensing pad, in order to transmit the pressure from the pressure sensing pad to the pressure transducer. Since the pressure transducer is located below the pressure sensing pad, the pressure does not need to be transmitted via a fluid tube over a larger distance. This can lead to a further improved measurement of the pressure from the surrounded body part. Furthermore, there is no tube anymore that might produce pressure marks on the surrounded body part, thereby allowing to make the wearing of the pressure measuring device even more comfortable, especially if the pressure transducer is integrated in the shell such that it does not protrude from the inner surface of the shell. It should be noted that the term "below" refers to an orientation of the pressure measuring device in which the pressure sensing pad is above the surface of the shell at the location where the pressure sensing pad is arranged.

The pressure measuring device can have a wired or a wireless data connection to a processor that is configured to determine a physiological parameter like blood pressure based on the pressure signal measured by the pressure transducer.

In an example the pressure measuring device comprises a height sensor configured to provide a height of the shell. The height sensor preferentially is configured to provide the height of the shell relative to a defined level, in particular relative to the phlebostatic axis. The height sensor can be any sensor being able to provide the height of the shell. The height sensor can include an accelerometer and/or a gyrometer. The height sensor can be integrated in the shell. In particular, the pressure sensing device can comprise a fluid-filled pressure sensing pad arranged on the inner surface of the shell, wherein the height sensor can be located below the pressure sensing pad. The measured height of the pressure measuring device can be considered when determining the physiological parameter based on the measured pressure signal. This can lead to a further improved accuracy of the determination of the physiological parameter like, for instance, the blood pressure.

As described above, the shell preferentially is a kinking-proof shell, wherein the kinking-proof shell is located (or sandwiched) between the pressure application element, i.e., e.g. a fluid bag, and the body part, when the pressure measuring device surrounds the body part for measuring the subject's physiological parameter. The kinking-proof shell - that is relatively stiff - avoids or at least significantly reduces the creation of wrinkles or kinks at the compression acting surface of the pressure application element, particularly of a fluid bag. Consequently, the measurement accuracy can be improved, since no wrinkles negatively influence the amplitude, form and reproducibility of the measured signal. In particular, since at least a part of the pressure sensing device is located between the shell and the body part, for instance, since a pressure sensing pad of the pressure sensing device can be located on the inner surface of the shell, a high accuracy of the signals measured by the pressure sensing device can be achieved. With such a configuration of the pressure sensing device, the shell does not absorb or attenuate the arterial pressure signal.

The kinking-proof shell preferably exhibits a stiffness notably larger than the stiffness of a wall of a fluid bag if the pressure application element comprises such a fluid bag. Preferably, the stiffness of the kinking-proof shell is chosen so as to ensure that no buckling of the kinking-proof shell will occur when pressure is applied to the body part by the pressure application element for measuring the subject's physiological parameter. At the same time, the kinking-proof shell should be flexible enough so as to allow the kinking-proof shell to reduce its inner diameter when pressure is applied by the pressure application element, for instance, by inflating a fluid bag of the pressure application element, for measuring the subject's physiological parameter.

Preferably, the kinking-proof shell is a stiffening component which is configured for ensuring stiffness of the pressure measuring device for pressure measurements. The kinking-proof shell may be the sole stiffening component of the pressure measuring device, the stiffening component exhibiting a structural solidity or strength and being configured for providing stiffness for or during pressure measurements.

Preferably, the kinking-proof shell is configured for interacting with the pressure application element such that by pressing the stiffening kinking-proof shell against the body part, the kinking-proof shell ensures appropriate stiffness of the pressure measuring system for pressure measurement. Such a stiffening kinking-proof shell allows for a straightforward design of the system. In particular, the stiffening kinking-proof shell may be pressed against the body part without interposition of any further stiffening element, i.e., between the stiffening kinking-proof shell and the body part preferentially there is no further element which provides any stiffening function.

Preferably, structural resistance of the pressure measuring device, especially against compression forces, is provided by the kinking-proof shell, especially exclusively by the stiffening kinking-proof shell. The stiffening kinking-proof shell may be the sole component of the pressure measuring device which exhibits structural resistance against compression forces.

Preferably, the kinking-proof shell has several functions like providing buckling strength and deformation resistance, especially with respect to compression forces. In other words, preferentially the kinking-proof shell exhibits a deformation resistance or dimensional stability which ensures sufficient stiffness of the pressure measuring device, especially in conjunction with a pressure applied by the pressure application element.

Preferably, the kinking-proof shell is a stiffening one-piece shell. Such a design allows for providing sufficient stiffness in a straightforward manner.

Preferably, the kinking-proof shell is dimensioned so as to overlap when surrounding the body part. In other words, the kinking-proof shell preferably completely surrounds the patient's body part, at least from the time when pressure is applied by the pressure application element. Thus, kinks or wrinkles can be avoided - or at least significantly reduced - along the whole circumference of the blood pressure measuring system. For example, the patient's body part may correspond to a patient's upper arm, leg or to a patient's wrist.

As kinks or wrinkles are avoided - or at least significantly reduced - due to the accommodation of the kinking-proof shell, overlapping portions of the shell, i.e., the lateral end portions of the shell, should be able to move or slide relatively to each other when its inner diameter is reduced due to the supply of pressurized fluid, preferably air, to, for instance, a fluid bag of the pressure application element. Therefore, the pressure measuring device, especially the kinking-proof shell, is preferably designed so that overlapping portions can easily slide relatively to each other. For example, surface portions of the shell that are in direct sliding contact with each other may exhibit a relatively low friction coefficient, e.g. by choosing the materials and/or the surface structures correspondingly. The term *"low friction coefficient"* in the context of the present invention refers to a friction coefficient of two flat surfaces of less than 0.5, preferably of less than 0.3, more preferably of less than 0.2, and even more preferably of less than 0.1.

In an example the shell, particularly the kinking-proof shell, is made from metal and/or plastic, in particular fiber-reinforced plastic. For example, the shell might be made from or coated with a thermoplastic material, preferably made from polyurethane or a polyolefin, more preferably made from polyethylene and/or polytetrafluoroethylene (PTFE) or coated with PTFE. For example, the shell might be made from a thermoplastic material which provides a surface on which adhesives may adhere durably. If the shell comprises fiber-reinforced plastic material, the fibers may be natural fibers, organic fibers or inorganic fibers.

Preferentially, the material and the shape, in particular the thickness, of the shell, especially of the kinking-proof shell, is chosen so that the shell, on the one hand, is stiff enough not to buckle when pressure is applied by the pressurization means, especially in case no additional flexible element is provided, and that the shell, on the other hand, is flexible enough so as to allow a reduction of its inner diameter when pressure is applied by the pressure application element. In particular, if the shell is substantially made from plastic material, in an example the shell exhibits a thickness within a range from 0.25 mm to 6 mm, more preferably within a range from 1 mm to 3 mm, even more preferably within range from 1.0 mm to 2.0 mm. For example, the shell may have a thickness of 1.5 mm, especially for adults. Or the shell may have a thickness of 0.5 mm, especially for infants and babies. In a preferred embodiment the shell is made from polyethylene (PE) having a thickness of 1.5 mm.

The thickness of the shell, particularly of the kinking-proof shell, may be reduced, preferably gradually, at the edge regions of the overlapping lateral end portions of the shell. Thus, the shell may have some kind of ramp-shaped form at its edge regions so as to promote relative sliding of the overlapping portions of the pressure measuring device. At these edge regions, the shell, especially the kinking-proof shell, may exhibit roundings and/or chamfers and/or taperings. Such a shape may facilitate sliding of one free end of the shell with respect to the other overlapping free end.

In an example, in order to avoid kinks on the one hand and to allow a reduction of the inner diameter on the other hand, the kinking-proof shell exhibits a modulus of elasticity of more than 50 MPa, more preferably within a range from 100 MPa to 10 GPa, even more preferably within a range from 200 MPa to 1 GPa.

The shell may exhibit a substantially cylindrical or conical configuration, so that the pressure measuring device can be easily applied to the patient's body part, preferably the patient's upper arm, without having to wrap the system around that body part. Preferably, the shell, however, exhibits a rather conical configuration so as to better adapt to the shape of the body part. A conical configuration may be provided by a spiral-shaped arrangement of the shell. In other words, the shell may be configured for twisting when the overlapping portions, i.e., the overlapping end portions, slide with respect to each other.

The pressure sensing device is preferably arranged so as to be at least partially located between the shell and the body part, preferably next to the body part, when the pressure measuring device surrounds the body part. The pressure sensing device may comprise, as mentioned above, a pressure sensing pad, which might be a gel or oil cushion or the like, in direct contact or in contact via the liner with the subject's body part.

In an example the pressure transducer or pressure sensor pad, i.e., the pressure sensing area on the inner surface of the shell, extends in a circumferential direction of the shell when the pressure measuring device surrounds the body part. Such an arrangement facilitates handling of the pressure measuring device. Correct or appropriate positioning of the pressure measuring device with respect to the body part may be facilitated.

Preferably at least the pressure application element and the shell, more preferably all components of the pressure measuring device, are connected to each other in such a way as to form a single unit. Such a configuration allows the pressure measuring device to be arranged as a single unit around the subject's body part for measuring the subject's pressure on the tissue, i.e., the tissue pressure, which in turn allows for an easy and fast application of the pressure measuring device and minimizes the risk of a faulty operation.

In a further aspect of the present invention an apparatus for determining a physiological parameter of a subject is presented, wherein the apparatus comprises a) the pressure measuring device for measuring the pressure signal of the subject over a time, wherein the measured pressure signal is indicative of blood pulsations, and b) a processor configured to determine the physiological parameter based on the measured pressure signal. Preferentially, the measured pressure signal comprises a plurality of pressure pulses. Moreover, the physiological parameter can be determined continuously, i.e., several subsequent physiological parameter measurements can be carried out, or it can be determined non-continuously, i.e., for instance one time.

The processor can be configured to determine, for a respective pressure pulse of the plurality of pressure pulses, at least one feature that characterizes the respective pressure pulse, to determine, for the respective pressure pulse, a physiological parameter determination value based on the determined at least one feature such that for several pressure pulses, which are present at different times, several physiological parameter determination values are determined, wherein the several physiological parameter determination values determined for the several pressure pulses and hence for several times form a physiological parameter determination curve, and to determine the physiological parameter based on the physiological parameter determination curve. For instance, the physiological parameter can be the blood pressure, the respiration rate or another physiological parameter.

The at least one feature can directly characterize the respective pressure pulse or indirectly characterize the respective pressure pulse. In the latter case the respective pressure pulse is processed for determining a feature determination pulse and the at least one feature is determined based on the determined feature determination pulse. This will be explained in more detail further below. The processor can be configured to determine, for each pressure pulse of all or of only some, for instance, at least 5 or at least 10, of the plurality of pressure pulses, the at least one feature that characterizes the respective pressure pulse.

The processor can be adapted to process the several physiological parameter determination values, which are obtained for the several pressure pulses, for obtaining a continuous physiological parameter determination curve. For instance, an interpolation can be applied and optionally also a smoothing.

In an embodiment, the processor is configured to, in order to determine for a respective pressure pulse the at least one feature, provide a feature determination pulse based on the respective pressure pulse, and determine at least one of the following features i) the difference (TPP) between the feature determination pulse's maximum systolic pressure (TPsys) and the feature determination pulse's pressure at the end-diastolic point (TPdia), ii) the area (TPA+.top50) enclosed by an upper part of the feature determination pulse, wherein a) the upper end of the upper part is at the feature determination pulse's maximum systolic pressure (TPsys) and b) the lower end of the upper part is between the feature determination pulse's maximum systolic pressure (TPsys) and a pressure value which corresponds to a mean (TPcl) of the measured pressure (TP), iii) the duration (t(pulse)) of the respective feature determination pulse, iv) the area (TPA/TPA.norm) of the respective feature determination pulse, and v) the width at half maximum (W50) of the respective feature determination pulse. It has been found that by using at least one of these features the determination of the physiological parameter can be further improved.

The feature determination pulse for a respective pressure pulse can be obtained, for instance, by subtracting a mean measured pressure (TPcl) from the respective pressure pulse. However, the feature determination pulse for a respective pressure pulse can also be determined in another way. It can also directly be the respective pressure pulse, i.e., the respective measured pressure pulse. If the feature determination pulse for a respective pressure pulse has been obtained by subtracting a mean measured pressure (TPcl) from the respective pressure pulse, for determining the area TPA+.top50 the pressure value, which corresponds to the mean (TPcl) of the measured pressure (TP), would be zero.

The difference (TPP) between the feature determination pulse's maximum systolic pressure and the feature determination pulse's pressure at the end-diastolic point preferentially is the difference between the maximum measured pressure and the minimum measured pressure of the respective pressure pulse, i.e., the difference between the tissue pressure (TP), i.e., the measured pressure on the skin, of the respective pressure pulse at a maximum systolic point and the tissue pressure (TP) at an end diastolic point of the respective pressure pulse. The duration t(pulse) of the respective feature determination pulse preferentially corresponds to the time difference between an end-diastolic point and the following end-diastolic point for the respective pressure pulse. Moreover, the area TPA is preferentially the area under the curve of the respective feature determination pulse from an end diastolic point to the following end diastolic point. The area can be a normalized area and is then named "TPA.norm". For instance, the area TPA can be normalized by scaling it to the difference between the maximum pressure and the minimum pressure of the respective feature determination pulse, in order to determine TPA.norm. The width W50 at half maximum corresponds to the width at 50 percent of the difference between the maximum pressure and the minimum pressure of the respective feature determination pulse. Thus, it is the width at 50 percent of the difference between the pressure of the feature determination pulse at the maximum systolic point and the pressure of the feature determination pulse at an end diastolic point.

In a preferred embodiment the processor is configured to determine the area TPA+.top50 enclosed by the upper part of the feature determination pulse such that the lower end of the upper part is at a pressure value which corresponds to half of the pressure distance (TPP+) between the feature determination pulse's maximum systolic pressure (TPsys) and the pressure value which corresponds to the mean (TPcl) of the measured pressure (TP).

Moreover, in a preferred embodiment the processor is configured to determine the physiological parameter determination values (TPWP_M) and hence form the physiological parameter determination curve (TPW_M-curve) only based on the feature being the difference (TPP) between the feature determination pulse's maximum systolic pressure (TPsys) and the feature determination pulse's pressure at the end-diastolic point (TPdia). Thus, in this embodiment, besides TPP, no other of the above mentioned features is used for determining the physiological parameter determination values. It has been found that this allows for a further increased accuracy of determining the physiological parameter with relatively low computational efforts.

In a further improved embodiment the processor is configured to determine the physiological parameter determination values (TPWP_M) and hence form the physiological parameter determination curve (TPW_M-curve) only based on a) the feature being the difference TPP between the feature determination pulse's maximum systolic pressure (TPsys) and the feature determination pulse's pressure at the end-diastolic point (TPdia) and b) the feature being the area TPA+.top50 enclosed by the upper part of the feature determination pulse. Thus, in this embodiment, besides TPP and TPA+.top50, no other of the above mentioned features is used for determining the physiological parameter determination values. It has been found that this allow for an even further increased accuracy of determining the physiological parameter, wherein still the computational efforts can be relatively low.

Preferentially, the processor is configured to determine a position of a maximum of the physiological parameter determination curve (TPW_M-curve) and to determine the physiological parameter, particularly the blood pressure, based on the determined position and the measured pressure (TP). In particular, the processor can be configured to determine the blood pressure based on the mean (TPcl) of the measured pressure (TP) at the determined position of the maximum. Determining the physiological parameter based on the determined maximum of the physiological parameter determination curve and depending on the measured pressure on the skin, i.e., depending on the tissue pressure, allows to further increase the accuracy of determining the physiological parameter, particularly the blood pressure.

Preferentially the processor is configured to provide a function which receives as input the at least one feature of a respective pressure pulse and which outputs a respective physiological parameter determination value which forms, together with the physiological parameter determination values determined for the other pressure pulses, the physiological parameter determination curve. The function has at least one parameter which can be determined by calibration, wherein by another measurement gold standard physiological parameters are determined very accurately and the at least one parameter is determined such that the apparatus yields the very accurately measured gold standard physiological parameters with high statistical precision and accuracy. For instance, by invasive means reference blood pressure values are determined very accurately and the at least one parameter is determined such that the apparatus yields the very accurately invasively measured blood pressure values with high statistical precision and accuracy. In particular, for the calibration measurement pairs of simultaneously recorded invasive and noninvasive blood pressure values from an adequate number of individuals in different hemodynamic conditions are used. It should be noted that the calibration is preferentially only carried out in a development phase, i.e., not during an actual blood measurement procedure. The calibration can be carried out separately for different shell sizes or for different groups of shell sizes. For instance, for different shell sizes or groups of shell sizes, different parameters of the function can be determined by calibration, i.e., for each shell size or for each group of shell sizes a respective at least one parameter can be determined.

In an embodiment the processor is configured to control the pressure applicator such that it increases the applied pressure with a first rate in a pre-measurement time period which is followed by the measurement time period in which the applied pressure is increased with a second rate, wherein the first rate is larger than the second rate. Thus, an applied pressure, at which the measurement time period should start, can be reached relatively fast, thereby allowing for a further reduction of the overall time needed for measuring the blood pressure.

In an embodiment the processor is configured to control the pressure actuator such that at the end of the pre-measurement time period the measured pressure is within a range of 15 to 30 mmHg. This ensures that during the measurement time period the pressure on the skin, i.e., the tissue pressure, is measured over a sufficiently large pressure range, which allows for an accurate and precise measurement of the physiological parameter.

It should be noted that the pressure at the end of the pre-measurement time is not the pressure which is applied in between two subsequent blood pressure measurements, particularly before the pre-measurement time period starts. This pressure could be named "attachment pressure" and preferentially does not exceed 15 mmHg.

In another aspect of the present invention a pressure measuring method for measuring pressure of a subject is presented, wherein the pressure measuring method comprises a) arranging a pressure measuring device as defined in any of claims 1 to 12 around a body part of the subject, b) applying pressure to the body part by a pressure application element of the pressurization device of the pressure measuring device, and c) measuring a pressure signal of the subject, wherein the measured pressure signal is indicative of blood pulsations, by the pressure sensing device of the pressure measuring device.

In a further aspect of the present invention a method for determining a physiological parameter of a subject is presented, wherein the method comprises a) applying the pressure measuring method as defined by claim 14, thereby measuring a pressure signal of the subject over a time, wherein the measured pressure signal is indicative of blood pulsations, and b) determining the physiological parameter based on the measured pressure signal by a processor.

In another aspect of the present invention a computer program for determining a physiological parameter of a subject is presented, wherein the computer program comprises program code means for causing the apparatus for determining a physiological parameter as defined by claim 13 to carry out the steps of the method as defined by claim 14.

It shall be understood that the pressure measuring device of claim 1, the apparatus for determining a physiological parameter of claim 13, the pressure measuring method of claim 14, the method for determining a physiological parameter of claim 15 and the computer program for determining a physiological parameter have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplary an embodiment of an apparatus for determining a physiological parameter of a subject, which comprises a pressure measuring device, in a situation in which a pressure cuff of the pressure measuring device is inflated,

Fig. 2 shows schematically and exemplary a shell of the pressure measuring device encasing an upper arm of the subject,

Fig. 3 shows schematically and exemplary the apparatus in a situation in which the pressure cuff is deflated,

Fig. 4 shows schematically and exemplarily the pressure measuring device,

Fig. 5 illustrates schematically and exemplarily a distance between the shell and a liner of the pressure measuring device,

Fig. 6 shows schematically and exemplarily a structure of a liner of a pressure measuring device,

Fig. 7 shows schematically and exemplarily a fluid bag of a pressure application element of the pressure measuring device in a flat, unrolled and non-filled condition,

Fig. 8 shows schematically and exemplarily a pressure transducer housing and its connection to a fluid tube and an electrical cable,

Fig. 9 shows schematically and exemplarily an interface between two different fluids,

Fig. 10 shows schematically and exemplarily a pressure sensing pad of a pressure sensing device of the pressure measuring device and a three-dimensional mesh within the pressure sensing pad,

Fig. 11 shows schematically and exemplarily the three-dimensional mesh in more detail,

Fig. 12 shows schematically and exemplarily locations for arterial marks relative to a center of a pressure sensing area,

Fig. 13 shows schematically and exemplarily a three-dimensional pressure sensing pad,

Fig. 14 shows schematically and exemplarily a shell of the pressure measuring device,

Fig. 15 shows schematically and exemplarily further details of the shell,

Fig. 16 shows schematically and exemplarily a bent proximal end portion of the shell shown in Fig. 15,

Fig. 17 shows schematically and exemplarily a bent distal end portion of the shell shown in Fig. 15,

Fig. 18 illustrates a tapering of a lateral end portion of the shell,

Fig. 19 illustrates schematically and exemplarily an overlapping of two lateral end portions of the shell,

Fig. 20 illustrates schematically and exemplarily a good alignment of lateral end portions of a shell,

Fig. 21 illustrates schematically and exemplarily a bad alignment between lateral end portions of a shell in a case of a relatively small cone angle of the upper arm,

Fig. 22 illustrates schematically and exemplarily a bad alignment of lateral end portions of a shell in a situation in which the cone angle of an upper arm is relatively large,

Fig. 23 shows schematically and exemplarily an exploded view of the pressure measuring device,

Fig. 24 shows schematically and exemplarily several components including pull tabs of the pressure measuring device,

Fig. 25 shows schematically and exemplarily a pull tab and an attachment element of the pressure measuring device,

Figs. 26 and 27 illustrate schematically and exemplarily an igloo-shaped housing for a fluid tube,

Fig. 28 shows schematically and exemplarily the fluid tube within the igloo-shaped housing,

Fig. 29 illustrates schematically and exemplarily an embodiment in which a pressure transducer is integrated in a shell of the pressure measuring device and in which optionally a height sensor is used,

Fig. 30 shows schematically and exemplary a measured tissue pressure and further values derived from the tissue pressure measurement,

Figs. 31 to 34 illustrate calculations of different features for a pressure pulse of the measured tissue pressure, and

Fig. 35 shows a flowchart exemplary illustrating an embodiment of a method for determining a physiological parameter of a subject.

## DETAILED DESCRIPTION OF EMBODIMENTS

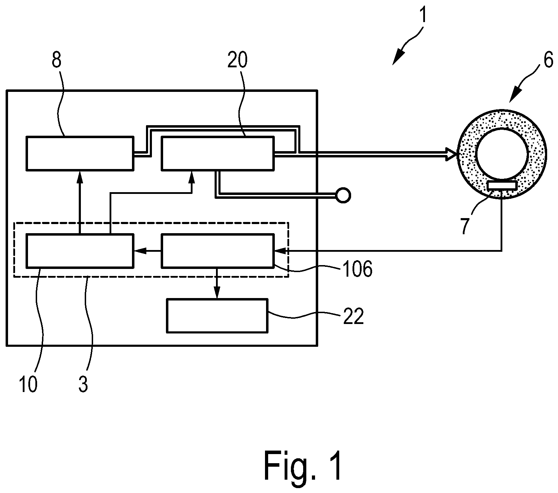

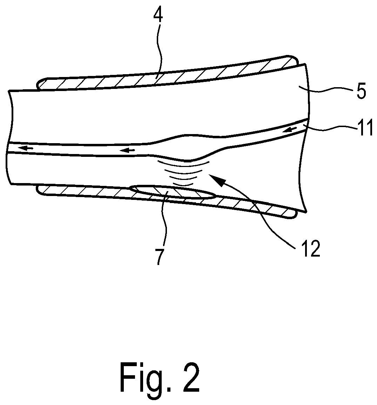

Fig. 1 shows schematically and exemplarily an apparatus 1 for determining a physiological parameter of a subject like the blood pressure. The apparatus 1 comprises a pressure measuring device 6 with a shell 4 which can be seen in Fig. 2 and which is configured to encase a part 5 of the subject, through which blood flows. In this embodiment the part 5 of the subject is an arm of the subject, wherein in Fig. 2 within the arm 5 a brachial artery 11 is shown and wherein the arrows within the brachial artery 11 indicate the flow direction of the blood away from the heart. The pressure measuring device 6 further comprises a pressure sensing device of which a part 7 is arranged on the inner side the shell 4 and which is configured to measure the pressure on the outer skin of the encased arm 5 of the subject. The measured pressure can also be regarded as being tissue pressure (TP). As indicated in Fig. 2, a pulse wave within the brachial artery 11 causes a pressure wave 12 which is transmitted to the pressure sensing part 7 of the pressure sensing device via the tissue of the arm 5. The shell 4 is not shown in Fig. 1 for clarity reasons. The shell 4 preferentially is a kinking-proof shell as described above.

The pressure measuring device 6 further comprises a pressure application element being a cuff which encloses the shell 4 and which is inflatable by using a pump 8 for applying, from outside the shell 4, pressure to the shell 4 and thereby to the encased arm 5 of the subject.

The apparatus 1 further comprises a processor 3 which is configured to control the pump 8 such that the pressure application element increases the applied pressure in a measurement time period extending till an end measurement time point and decreases the applied pressure in a following post-physiological-parameter-measurement time period and that the pressure sensing device with the pressure sensing part 7 measures the pressure on the skin, i.e., the tissue pressure TP, at least during the measurement time period. Moreover, the processor 3 is configured to control the pump 8 such that it increases the applied pressure - via the cuff - with a first rate in a pre-measurement time period which is followed by the measurement time period in which the applied pressure is increased with a second rate, wherein the first rate is larger than the second rate. The control of the measurement device such that the cuff is inflated and hence the applied pressure is increased is illustrated in Fig. 1, i.e., the bold arrows indicate the inflation situation.

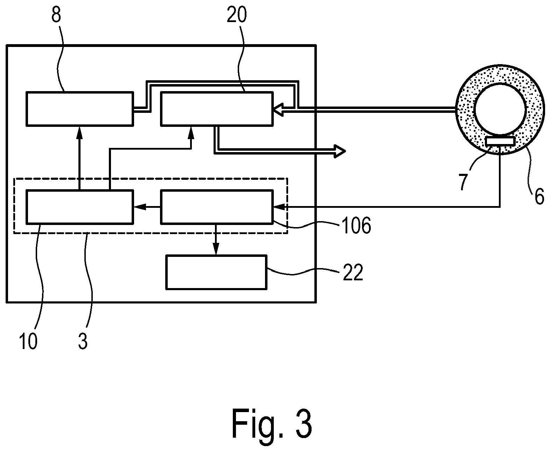

The apparatus 1 comprises a valve 20 which, when the valve 20 is opened, allows the compressed air within the apparatus 1 to leave the apparatus into the surrounding atmosphere for deflating the cuff. In Fig. 3 this deflation situation, in which the pump 8 is switched off, is indicated by the bold arrows.

In the pre-measurement time period, which could also be regarded as being a fast inflation period, the processor 3 controls the apparatus 1 such that the valve 20 is closed and the pump 8 inflates the cuff 6 with the larger first rate. In the following measurement time period the processor 3 also controls the apparatus 1 such that the valve 20 is closed, but the pump 8 is controlled such that the inflation of the cuff 6 is continued with the lower second rate. The measurement time period could therefore also be regarded as being a slow inflation period.

The processor 3 can be regarded as comprising a controlling part 10 for controlling the pump 8 and the valve 20 and a processing part 106, which especially is configured to carry out some calculations that will be explained further below. The apparatus 1 can also comprise a display 22 for displaying the determined physiological parameter like the blood pressure.

In the following the pressure measuring device 6 will be described in more detail.

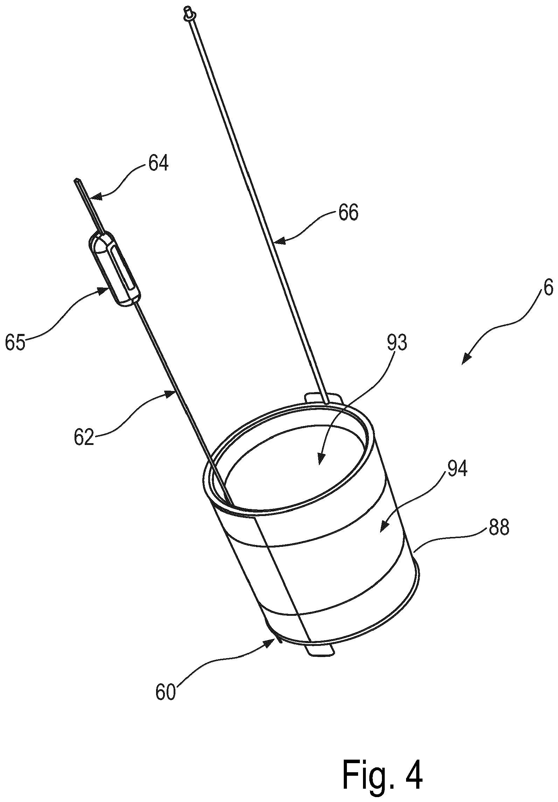

Fig. 4 shows schematically and exemplarily some components of the pressure measuring device 6. The pressure measuring device 6 comprises an elastic liner 93 at least partly arranged inside of the shell 4, wherein in Fig. 4 the shell 4 is not visible, because its inner surface and the edges of the shell 4, which in Fig. 4 are top edges and bottom edges, are covered by the liner 93. In an embodiment, there is free space in between the liner 93 and the inner surface of the shell 4, when the pressure measuring device 6 is substantially cylindrical or conical and does not surround the body part 5 as shown in Fig. 4, wherein the maximum distance between the liner 93 and the inner surface of the shell 4 is within a range of 0.5 cm to 2.0 cm. In a preferred embodiment this maximum distance is 1.0 cm.

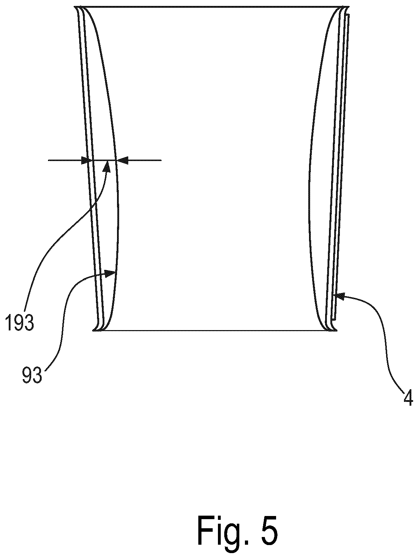

Fig. 5 schematically and exemplarily illustrates the maximum distance 193 between the liner 93 and the inner surface of the shell 4. Preferentially, the liner 93 is attached to the shell 4 at the distal and proximal edges of the shell 4. Moreover, the maximum distance between the liner 93 and the surface of the shell 4 preferentially is at the mid location between the proximal and distal edges of the cuff 4. Furthermore, preferentially in between the proximal and distal edges of the shell 4 the liner 93 is free on the inner surface of the shell 4 and hence, for instance, not stuck to the inner surface of the shell 4. The liner 93 preferentially comprises antimicrobial material. Thus, it can be equipped with an antimicrobial material or coating.

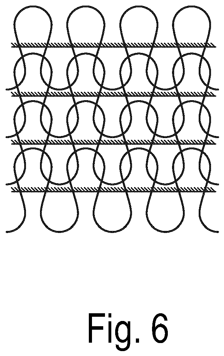

If the liner 93 were too loose, this could result in wrinkles which can cause pressure marks on the subject's body part 5. For this reason, the liner 93 preferentially is of a structure with a relatively small thickness such that air inclusions by the fabric can be reduced. For instance, the thickness can be in the range from 0.3 mm to 0.5 mm. Moreover, the structure preferentially is such that it allows extension from a minimal dimension (lowest circumference) to a maximal dimension (largest circumference) without significant increase of tension and returns to its minimal dimension (lowest circumference) after having been stretched to the maximal dimension (largest circumference). In particular, the liner 93 is of the weft knit type (two yards in single knit jersey structure and similar), as schematically and exemplarily indicated in Fig. 6. Moreover, preferentially the liner 93 is made of a material comprising a percentage of nylon, 70D within the range from 80% to 90%, and a percentage of nylon Spandex, 40D within a range from 10% to 20%. In a preferred embodiment the material comprises 86% nylon, 70D and 14% nylon Spandex, 40D.

Thus, the liner 93, particularly the liner fabric, has an elasticity and shape of knits which allow a flexible variation of the circumference of the body part 5 from a smallest circumference to a largest circumference without generating wrinkles and without causing tension that leads to pressure applied to the surrounded body part above a permissible pressure threshold. The permissible pressure threshold is preferentially defined such that, if the applied pressure has a value being equal to or below the value of the permissible pressure threshold, no venous congestion develops. In an example this pressure threshold is within a range from 20 mmHg to 40 mmHg and in an embodiment it is 30 mmHg.

The liner 93 can be in accordance with class II of the FDA standard for compression hosiery. It has been found that particularly in this case the liner 93 is flexible enough to be extended to a maximum circumference, wherein still there are no wrinkles when used with the smallest circumference. The ratio of the smallest circumference relative to the largest circumference of the liner 93 preferentially is within a range from 60% to 80% and further preferred within a range from 70% to 75%. In particular, different sizes of pressure measuring devices 6 and hence of shells 4 with liners 93 can be provided, wherein in an embodiment for a first pressure measuring device the smallest circumference of the liner 93 is 190 mm and the largest circumference is 270 mm, for a second pressure measuring device the smallest circumference of the liner is 250 mm and the largest circumference is 340 mm, for a third pressure measuring device the smallest circumference of the liner 93 is 300 mm and the largest circumference is 400 mm and for a fourth pressure measuring device the minimum circumference of the liner 93 is 320 mm and the largest circumference is 430 mm. Also other pressure measuring devices can be provided, especially pressure measuring devices for preterm infants can be provided, wherein the circumference might be, for instance, in a range from 30 mm to 50 mm. Preferentially, also in this case the ratio between the smallest circumference and the largest circumference is within a range from 60% to 80% and further preferred within a range from 70% to 75%. The circumference values and circumference percentages preferentially refer to the medial position in between the proximal and distal edges of the shell 4. The liner 93, i.e., the liner fabric 93, is arranged between the skin of the body part and the shell 4 for preventing pinching of the skin and of hairs. However, besides causing pressure marks due to wrinkles, generally a liner between the skin and the pressure sensing part 7 of the pressure sensing device on the inner surface of the shell 4 could dampen the tissue pressure waves and cause signal loss. For this reason, the liner 93 has yarn threads that are as strong as possible, but flexible, and a structure, i.e., a knitting, which allows for a relatively small thickness of the liner 93 and which reduces dampening air inclusions when applied to the body part 5.

The yarns of the liner 93 preferentially have a thickness within the range from 0.10 to 0.25 mm, further preferred within the range from 0.13 to 0.23 mm. The liner 93 has been produced preferentially by knitting. The thickness of the liner 93 preferentially is within the range from 0.3 to 0.5 mm. The elasticity of the liner material 93 can be such that it can be elastically extended by a percentage value within the range from 40% to 50%, preferentially without applying a significant pressure on the body part 5 and preferentially without plastic deformation and overexpansion. However, the elasticity or stretchability can also be larger than 50%. In an example, a significant pressure is not applied on the body part 5, if the applied pressure is below a pressure threshold being within a range from 20 mmHg to 40 mmHg, wherein in an embodiment this pressure threshold is 30 mmHg.

The liner 93 hence preferentially is a textile layer. The liner 93 can cover the inner surface and optionally also the outer surface of the shell 4. In particular, the liner 93 is arranged between the shell 4 and the body part 5 and it can also be arranged between a fluid bag 88 and the shell 4. Thus, any friction due to relative motion between the shell 4 and the body part 5 or the fluid bag 88 may be minimized. The liner 93 might surround both sides of the shell 4 in a sock-like manner, i.e., like a stocking.

Referring again to Fig. 4, the pressure application element 60, i.e., the pressure cuff, includes the fluid bag 88 and an attachment band 94 which surrounds the fluid bag 88 and which is configured to attach a casing 65, which encases a housing of a pressure transducer, to the fluid bag 88 via, for instance, a hook-and-loop fastener or another solvable connection. The fluid bag 88 is fillable with fluid for applying the pressure to the body part 5 via the shell 4. For filling the fluid bag 88 with the fluid and for releasing the fluid from the fluid bag 88 the fluid bag 88 can be connected to the pump 8 and the valve 20 via a tube 66.

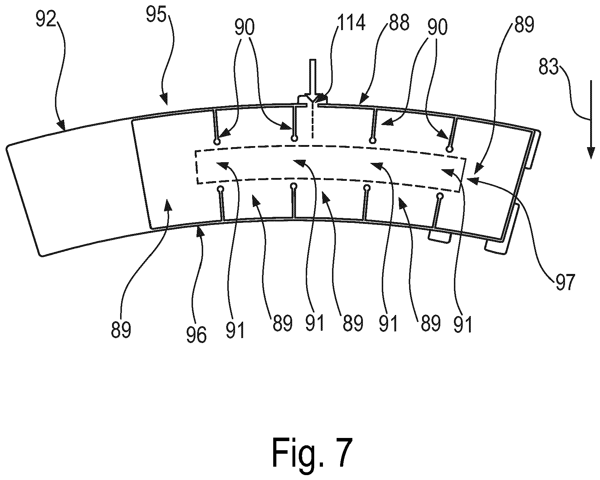

As illustrated in Fig. 7, the fluid bag 88 comprises compartments 89 which are separated by compartment walls 90 with openings 91 such that fluid can flow from compartment 89 to compartment 89, when fluid enters the compartments 89 of the fluid bag 88 via an opening 114. Within the fluid bag 88, an inlay 97 is arranged that keeps the gaps between the walls, i.e., the openings 91, open and guarantees the flow of the fluid between all compartments. The compartment walls 90 are perpendicular to the proximal and distal edges 95, 96 of the fluid bag 88. Moreover, the respective opening 91 in the respective compartment wall 90 is arranged in the center of the respective compartment wall 90. Preferentially, the respective opening 91 in the respective compartment wall 90 has a height within a range from 35% and 45% of the height of the fluid bag 88, wherein the height of the fluid bag 88 is measured as a distance between the proximal and distal edges 95, 96 of the fluid bag 88.

Moreover, the fluid bag 88 has an end portion 92 in which no fluid is flowable. This end portion 92 has an area within a range from 20% to 25% of the entire area of the fluid bag 88, if the fluid bag 88 is in a flat, unrolled and non-filled condition as shown in Fig. 7. The end portion 92 and the other, opposing lateral end portion of the fluid bag 88 preferentially comprise fastening means like hook-and-loop fastener elements or another solvable connection for allowing the end portions to fasten to each other when they overlap, after the pressure measuring device has been wrapped around the subject's body part.

The fluid bag 88 has a shape that follows a curve element, if the fluid bag 88 is in the flat, unrolled and non-filled condition as shown in Fig. 7. The fluid bag 88 has a substantially elongate shape, wherein the elongated shape follows the curve element, when the fluid bag is in the flat, unrolled and non-filled condition. The end portion 92, in which no fluid is flowable, is located at a lateral end portion of the fluid bag 88, wherein the lateral direction is defined as being substantially perpendicular to the proximal-distal direction 83.

The areas covered by the compartment walls 90 and the end portion 92 of the fluid bag 88 can be regarded as being deactivated areas, because these areas are not active when applying pressure to the body part 5. These deactivated areas might be created by welding. For instance, the compartment walls 90 and/or the end portion 92 can be realized as welding seams. The deactivated areas of the fluid bag 88 lead to a more uniform distribution of the pressure applied to the body part 5 via the shell 4. Especially if the shell 4 has a conical shape, as it is preferentially the case, a known fluid bag without the deactivated areas unwinds from the shell and thereby causes the non-uniform distribution of the applied pressure. This adverse effect can be reduced by the deactivated areas 90, 92 of the fluid bag 88.

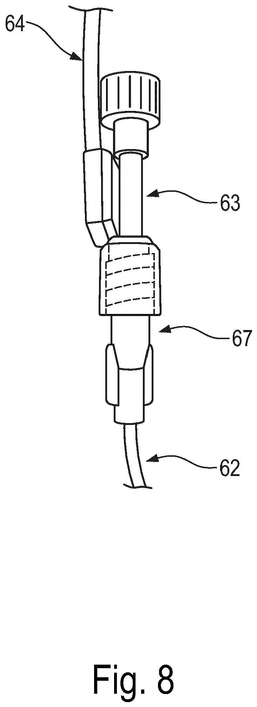

The pressure transducer housing 63 within the casing 65 is schematically and exemplarily shown in Fig. 8. The pressure transducer housing 63 is fluidly connected with a pressure sensing pad 61, which is arranged in a pressure sensing area 72 on the inner surface of the shell 4, via a tube 62. The fluid-filled tube 62 hence is configured for guiding pressure from the pressure sensing pad 61 to the pressure transducer, which preferentially is a differential pressure transducer (DPT), within the pressure transducer housing 63.

In this embodiment, the pressure sensing pad 61 and the tube 62 are filled with a first fluid and the pressure transducer housing 63 is filled with a second fluid, wherein the first and second fluids have different viscosities. In particular, the first fluid has a lower viscosity than the second fluid. For instance, the first fluid can be silicone oil and the second fluid can be glycerol. In a preferred embodiment the viscosity of the first fluid is at least 100 times smaller than the viscosity of the second fluid.

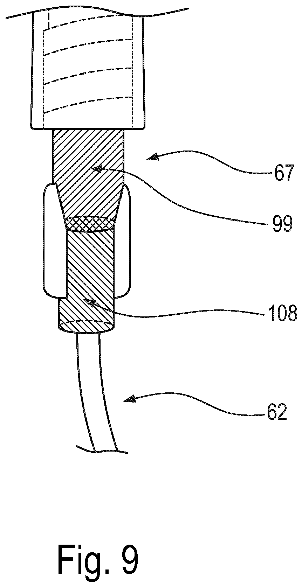

As can be seen in Fig. 8, the pressure transducer housing 63 is connected to the tube 62 via a connector 67 being, for instance, a Luer connector. The measured pressure signal is guided to the processor 3 via a cable 64. Fig. 9 schematically and exemplarily illustrates the interface between the first fluid 108 and the second fluid 99 in the connector 67.

The pressure transducer housing 63 preferentially is filled with a fluid being compatible with the material of the pressure transducer housing 63 and the material of the pressure transducer itself. In an example, the pressure transducer housing is made of or comprises polycarbonate and the pressure transducer comprises silicon-based parts. Thus, in an example, the fluid within the pressure transducer housing 63 is compatible with polycarbonate and silicon. Especially for this reason, in an example the fluid within the pressure transducer housing 63 is glycerol.

The fluid within the pressure sensing pad 61 and the tube 62 preferentially has a low viscosity, in order to provide a good pressure transmission from the surface of the pressure sensing pad 61 to the pressure transducer housing 63. For this reason, a low viscosity fluid is preferred like silicone oil. It is further preferred that the fluid within the pressure sensing pad 61 has no or only a very low permeability through the wall or foil of the pressure sensing pad 61. Since silicone oil does not vapor or vaporizes very slowly, i.e., generally over years, through a wall of foil of the pressure sensing pad 61, also for this reason silicone oil is preferentially used in the pressure sensing pad 61. If the permeability of the foil or wall of the pressure sensing pad 61 is relatively low for the fluid, it can be prevented that the pressure sensing pad 61 dries out during shelf life.

The fluids within the pressure transducer housing 63, the pressure sensing pad 61 and the tube 62 connecting the pressure transducer housing 63 and the pressure sensing pad 61 are preferentially liquids. Using two liquids with largely different viscosity prevents mixing and builds a natural stable interface between the two liquids, wherein this interface can be like a membrane and wherein this interface also allows for a good transmission of the pressure from the liquid in the tube 62 to the liquid in the pressure transducer housing 63.

The pressure sensing pad 61 is preferentially filled with a fluid having a fluid thickness, in a flat condition of the pressure sensing pad 61, within a range from 0.90 mm to 1.10 mm. Preferentially, the fluid thickness is 1.07 mm in the flat condition of the pressure sensing pad 61. The fluid thickness is preferentially measured as the thickness of the fluid between the foils or walls of the pressure sensing pad 61, when the pressure sensing pad is in a flat condition. In use, the foils or walls, which are relevant here, are the foils or walls being parallel to the inner surface of the shell 4.

It has been found that this fluid thickness can prevent a pressure offset in a mounted shape at a relatively small circumference and that it can also minimize wrinkles or the likelihood of separation in fluid compartments over the area of the pressure sensing pad 61. In particular, it has been found that the fluid thickness of 1.07 mm is an optimal value for different sizes of the pressure measuring device 6. For instance, four pressure sensing pads had different active areas, i.e., areas filled with fluid, of 2114.0 mm$^2$, of 2796.6 mm$^2$, of 3192.1 mm$^2$ and of 3648.6 mm$^2$, wherein in each of these four cases the optimal fluid thickness was in a range from 0.90 mm to 1.10 mm and was particularly 1.07 mm. Generally, under- or overfilling of the fluid can influence the measurement performance, for instance, can lead to a poor signal amplitude or to a pressure offset due to an increasing internal pressure. As mentioned above, it has been found that a fluid thickness within the range from 0.90 mm to 1.10 mm, especially of 1.07 mm, can lead to an improved measurement performance.

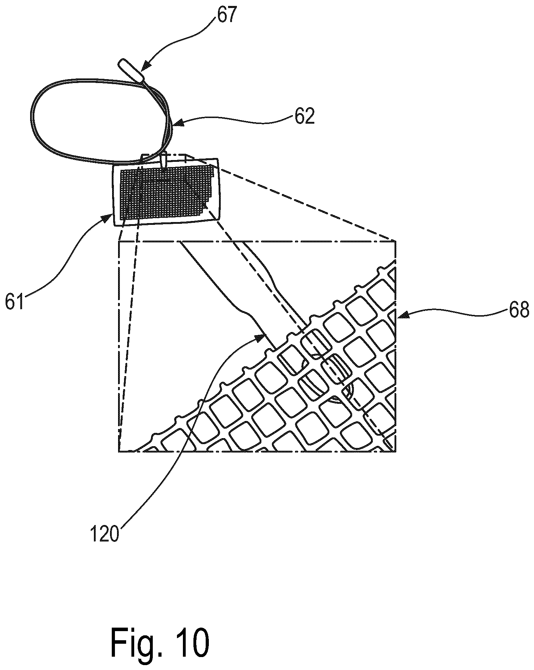

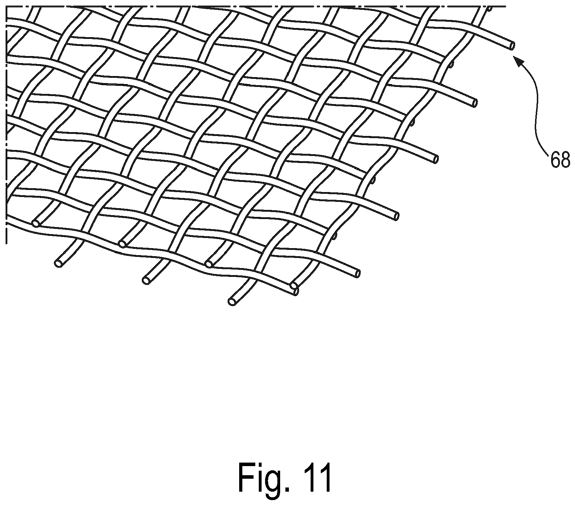

In an example a mesh 68 is arranged within the pressure sensing pad 61 as schematically and exemplarily illustrated in Fig. 10. Preferentially, the mesh 68 is a three-dimensional mesh. The three-dimensional mesh 68 generates fluid channels within the pressure sensing pad 61 and it can reduce the likelihood of a closure of the end 120 of the tube 62 that is arranged within the pressure sensing pad 61. Moreover, the three-dimensional mesh 68 can reduce the likelihood of forming larger non-fluid filled compartments within the pressure sensing pad 61. The term "three-dimensional mesh" means "woven structure". A corresponding three-dimensional mesh, i.e., woven structure, is schematically and exemplarily shown in Fig. 11.

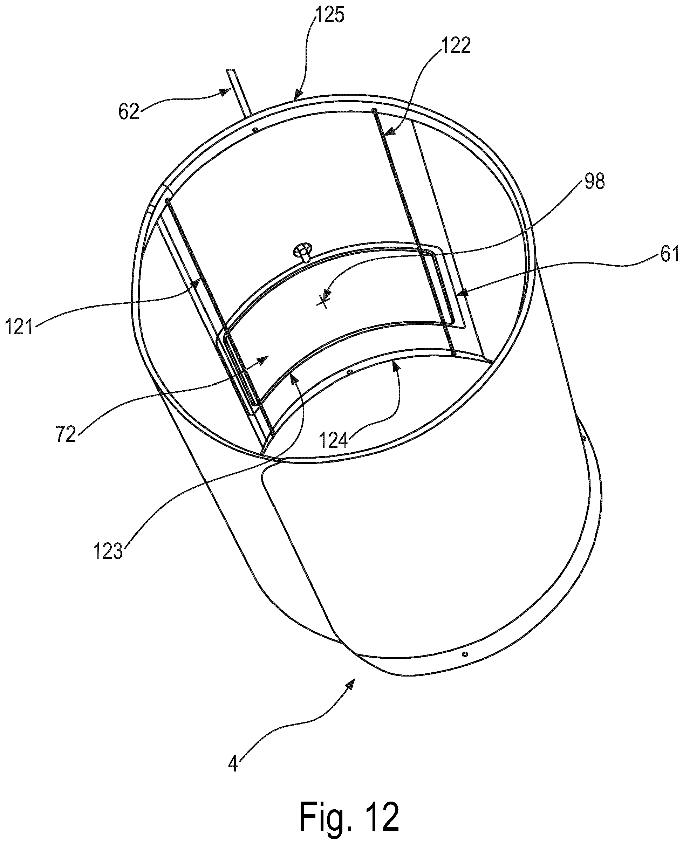

The pressure sensing pad 61 is located within a pressure sensing area 72 on an inner surface of the shell 4 as schematically and exemplarily illustrated in Fig. 12. The pressure sensing area 72 is identical to the active area of the pressure sensing pad 61, i.e., the dimensions of the active area of the pressure sensing pad 61 define the pressure sensing area 72. The shell 4 comprises marks 121, 122 having an angular offset within a range from 35° to 45° relative to the location of the center 98 of the pressure sensing area 72. Preferentially, this offset is 40°. This angular offset can be clockwise or counter-clockwise and is with respect to an imaginary rotational axis coinciding with the axis of the cylindrical or conical shape illustrated in Fig. 12. It should be noted that preferentially the shell 4 is not cylindrical, but it is conical, especially slightly conical.

When arranging the pressure measuring device 6 on an upper arm of a subject, it should be arranged such that the mark 121 coincides with the brachial artery, if the upper arm is the left upper arm, and such that the mark 122 coincides with the brachial artery, if the upper arm is the right upper arm. Using the arterial marks 121, 122 for positioning the pressure measuring device 6 on the left upper arm or the right upper arm improves the quality of the measured pressure signal, i.e., of the measured tissue pressure waveforms, and tolerates slight misalignments best.

It should be noted that Fig. 12, like Fig. 4, only shows some parts of the pressure measuring device 6 and not all parts for clarity reasons. The arterial marks 121, 122 are provided such that they are visible to the medical personnel when the pressure measuring device 6 is assembled completely. The arterial marks 121, 122, of which indeed only the angular locations are shown in Fig. 12, can be provided on the liner 93 or on another part of the pressure measuring device 6, which is visible to the medical personnel, if the pressure measuring device 6 is assembled completely and used by the medical personnel.

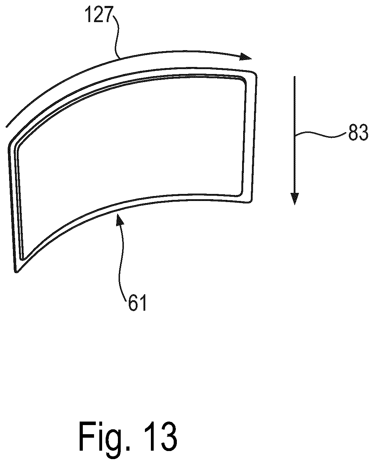

The pressure sensing pad 61 preferentially is designed three-dimensionally as schematically and exemplarily illustrated in Fig. 13. It should be noted that Fig. 13 shows the three-dimensionality of the pressure sensing pad 61, without further components of the pressure measuring device 6 like the tube 62 that is to be introduced into the pressure sensing pad 61 via an opening, for clarity reasons. By using a three-dimensional pressure sensing pad 61, in contrast to using a two-dimensional pressure sensing pad, the likelihood of creating folds on the inner surface of the shell 4 can be reduced. This allows for a further improved measurement of the pressure from the subject's body part.

The term "three dimensional" with respect to the pressure sensing pad preferentially means that the pressure sensing pad is formed by two layers, i.e., an outer layer and an inner layer, wherein the inner layer has a smaller dimension than the outer layer in one or two spatial directions. Preferentially, at least in the circumferential or lateral direction 127 the inner layer is smaller than the outer layer as schematically and exemplarily illustrated in Fig. 13. Also in the proximal-distal direction the inner layer can be smaller than the outer layer as also schematically and exemplarily illustrated in Fig. 13. In contrast to this, the term "two-dimensional" in connection with a pressure sending pad preferentially means that the inner layer and the outer layer have the same size and are congruent. Since the three-dimensional pressure sensing pad has an outer layer being larger than the inner layer, especially in the lateral or circumferential direction 127, the likelihood of wrinkles can be significantly reduced.

The inner and outer layers are preferentially foils. Moreover, the edges of the inner layer are attached, for instance, welded, to the outer layer.

The terms "inner layer" and "outer layer" are defined relative to the cylindrical or conical shell. The inner layer is directed away from the shell, i.e., towards the inner area enclosed by the shell, and the outer layer is directed towards the shell, i.e., away from the inner area enclosed by the shell.

Referring again to Fig. 12, the distance between an outer distal edge 123 of the pressure sensing area 72 and an outer distal edge 124 of the shell 4 is within a range from 15% to 20% of the entire height of the shell 4 from its outer distal edge 124 to its outer proximal edge 125 and/or the distance between the outer distal edge 123 of the pressure sensing area 72 and the outer distal edge 124 of the shell 4 is within a range from 20 mm to 30 mm. In a preferred embodiment, the distance between the outer distal edge 123 of the pressure sensing area 72 and the outer distal edge 124 of the shell 4 is 17%, further preferred 17.2%. Moreover, in a preferred embodiment, the distance between the outer distal edge 123 of the pressure sensing area 72 and the outer distal edge 124 of the shell 4 is 25 mm. These distances are especially used, if the pressure sensing area 72 has a dimension between 65 mm and 75 mm in the lateral direction 127, which is exemplarily indicated in Fig. 13, and a dimension between 35 mm and 45 mm in the proximal-distal direction 83. Preferentially, the pressure sensing area has a dimension of 70 mm in the lateral direction 127 and a dimension of 40 mm in the proximal-distal direction 83.

It should be noted that the pressure sensing area 72 preferentially is the area which really is active regarding the pressure measurement. For instance, if an outer surrounding edge of a pressure sensing pad 61 does not comprise fluid and hence is not pressure-sensitive, this outer surrounding edge of the pressure sensing pad does not belong to the pressure sensing area 72, but only the fluid-filled area of the pressure sensing pad 61 defines the pressure sensing area.

It has been found that the above described distances between the outer distal edge of the pressure sensing area and the outer distal edge of the shell lead to an improved pressure signal transmission from the body part of the subject to the pressure sensing pad 61. Due to the preferred conical shape of the pressure measuring device 6, particularly of the shell 4, the occlusion of the artery happens more distally, wherein for this reason it is preferred that the location of the pressure sensing area 72 is closer to the distal edge of the shell 4 than to the proximal edge of the shell 4.

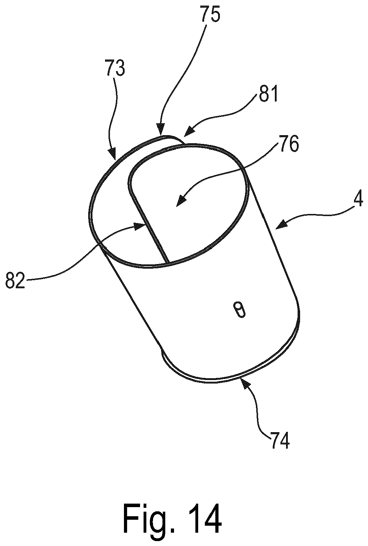

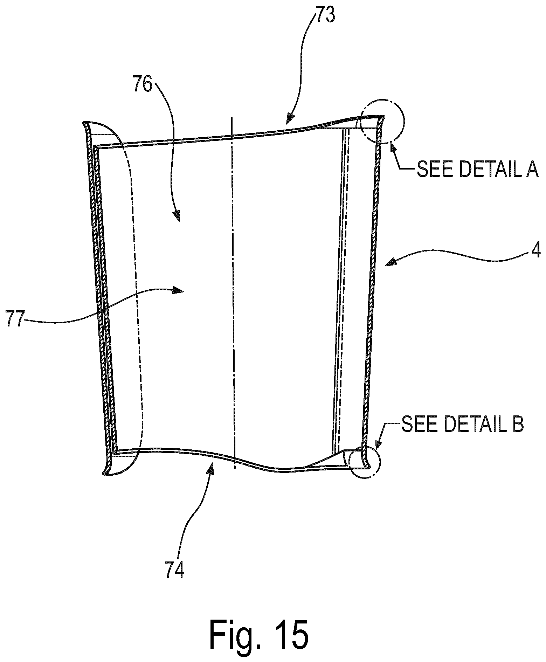

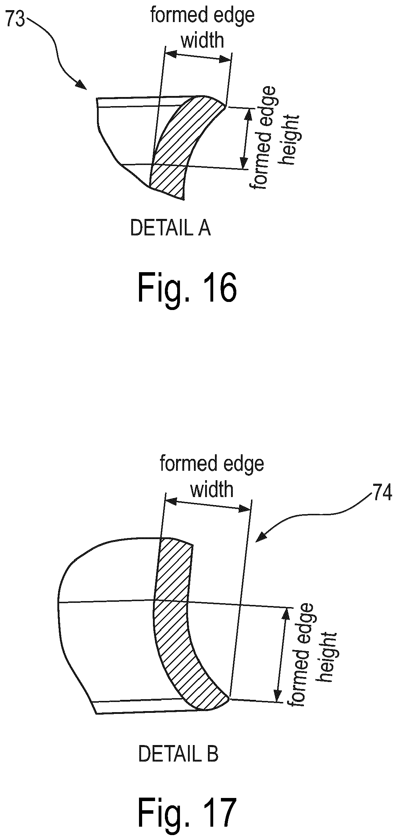

As schematically and exemplarily illustrated in, for instance, Fig. 14, the shell 4 comprises proximal and distal end portions 73, 74 and two lateral end portions 75, 76, wherein the two lateral end portions 75, 76 overlap when the shell 4 is in its cylindrical condition, which in fact is a slightly conical shape, as shown in Fig. 14. The proximal and distal end portions 73, 74 are at least partly bent away from the body part 5, when the pressure measuring device 6 surrounds the body part 5, as schematically and exemplarily illustrated in Figs. 15, 16 and 17, wherein Fig. 16 illustrates the detail A shown in Fig. 15 and Fig. 17 illustrates the detail B shown in Fig. 15.

Preferentially, the height of the bent distal end portion 74 is within a range from 6.3 mm to 7.3 mm and further preferred is within a range from 6.37 mm to 7.25 mm and the width of the bent distal end portion 74 preferentially is within a range from 3.2 mm to 5.0 mm, wherein the height is measured in the proximal-distal direction and the width is measured in a radial direction and wherein the radial direction refers to the radius of the cylindrical configuration, which in fact is a slightly conical configuration, of the shell 4 as illustrated in Fig. 14.

Moreover, preferentially, the height of the bent proximal end portion 73 is within a range from 3.9 mm to 4.5 mm, especially within a range from 3.96 mm to 4.47 mm, and the width of the bent proximal end portion 73 is within a range from 2.3 mm to 4.0 mm, wherein again the height is measured in a proximal-distal direction and the width is measured in a radial direction.

The bent proximal and distal end portions 73, 74 can be regarded as forming flares, i.e., widening shapes, which reduce the likelihood of pressure marks on the subject's body part when surrounded by the pressure measuring device 6. They also increase the comfort for the subject, because there is less skin trauma. The bent proximal and distal end portions 73, 74 also can be softer than the main part 77 of the shell 4 or as the remaining part of the shell 4, in order to further decrease the likelihood of generating pressure marks on the subject's body part 5 and in order to further improve the comfort for the subject.

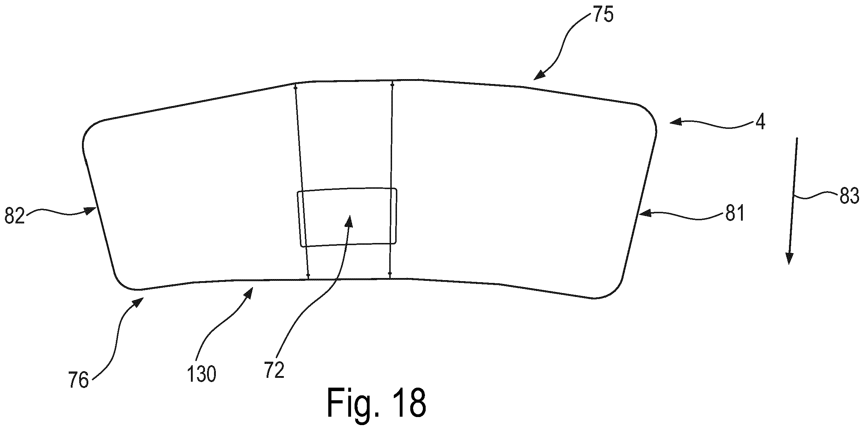

Fig. 18 shows schematically and exemplarily the shell 4 in a flat condition. As can be seen in Fig. 18, the lateral end portion 76 is tapered, wherein the tapering of the lateral end portion 76 starts between the outer lateral edge 82 of the lateral end portion 76 and the location of the pressure sensing area 72. The tapering of the shell 4 in the overlapping area, i.e., the tapering of the lateral end portion 76, can prevent that the shell differs from the conical shape of the cuff 60 at relatively small circumferences or relatively large circumferences of the body part 5 of the subject. It should be noted that, although not shown in Fig. 18 for clarity reasons, the shell 4 comprises an opening for accommodating the tube 62 that is connected to the pressure sensing pad arranged in the pressure sensing area 72.

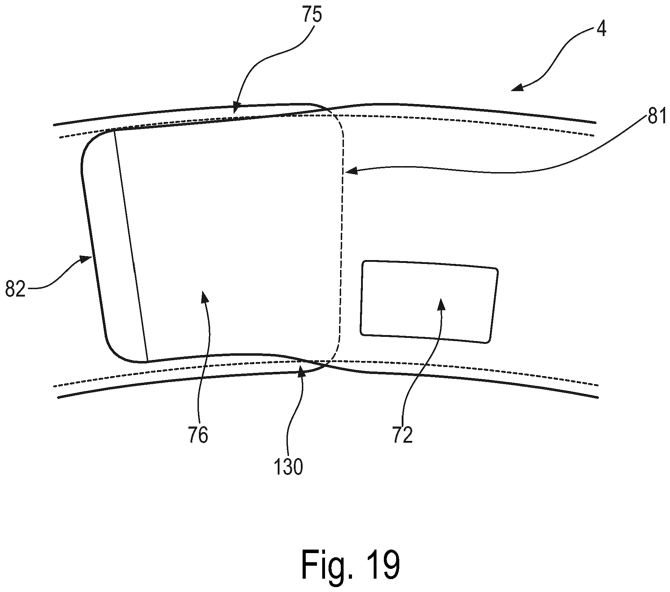

The tapering of the lateral end portion 76 is also illustrated in Fig. 19 which shows the tapered lateral end portion 76 overlapped and arranged on the opposing non-tapered other lateral end portion 75. Moreover, the lateral outer edge of the end portion 76 can also comprise a flattened section, i.e., a section having a smaller thickness than the other part of the end portion 76, in order to have a smaller step caused by the lateral outer edge of the end portion 76 on the inner surface of the other lateral end portion 75, if the shell is in the cylindrical or conical configuration.

As can be seen especially in Fig. 15, preferentially the lateral tapered end portion 76 does not comprise the flares, i.e., the proximal and distal end portions 73, 74 of the tapered lateral end portion 76 are not bent away from the inside of the pressure measuring device 6.

As can be seen in Figs. 18 and 19, the tapering 130 starts adjacent to or besides the pressure sensing area 72 in the width direction or lateral direction of the shell 4. The tapering can assure an equal distance between the distal edge of the shell and the distal edge of the pressure sensing area for different cone angles of the body part of different subjects. This allows to more reliably measure the pressure from the body part for different subjects having different cone angles of the body part.

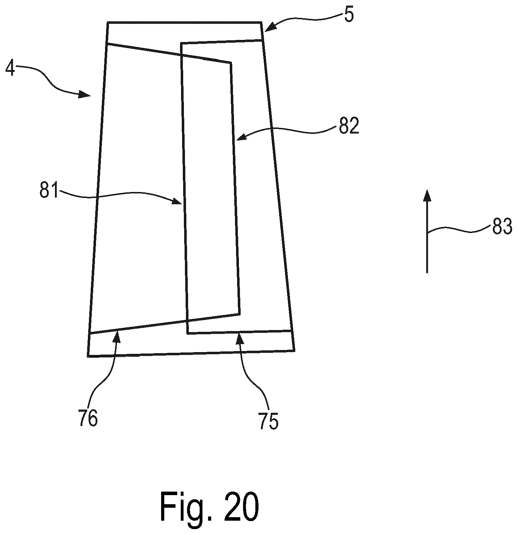

The problems, which can be solved by the tapering of the lateral end portion 76, will be further described with reference to Figs. 20 to 22 in the following.

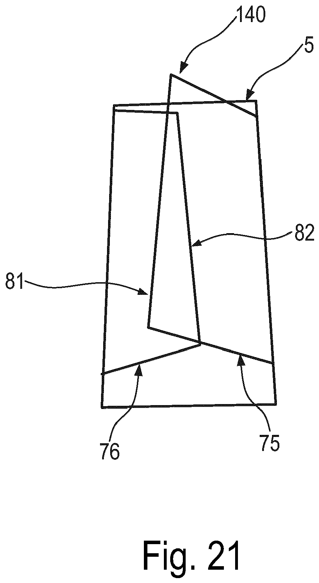

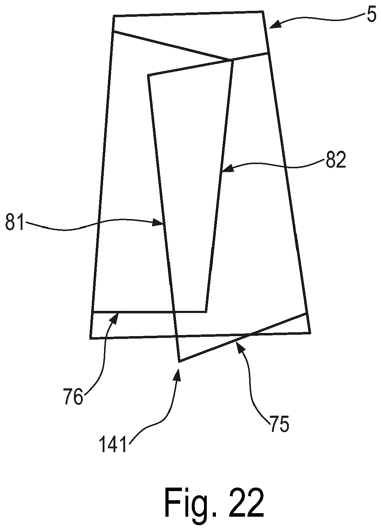

A pressure measuring device normally is designed for an expected cone angle of, for instance, an upper arm of a subject. If the actual upper arm 5 really has the expected cone angle, the lateral end portions 75, 76 with the outer lateral edges 81, 82 are well aligned and there is substantially no protrusion in the proximal-distal direction 83. This is schematically and exemplarily illustrated in Fig. 20. If the actual cone angle of the body part 5 is much smaller than the expected cone angle, as schematically and exemplarily illustrated in Fig. 21, the lateral end portions 75, 76 and also the outer lateral edges 81, 82 are not well aligned anymore and a part 140, which might be regarded as being an inner tongue, protrudes in the proximal-distal direction. Moreover, in the configuration shown in Fig. 21, the pressure sensing area might not be properly arranged on the body part 5. Fig. 22 schematically and exemplarily illustrates a situation in which the actual cone angle of the body part 5 is larger than the expected cone angle, which leads to a misalignment of the lateral end portions 75, 76 and the outer lateral edges 81, 82, with a resulting protruding part 141 of the lateral end portion 75. Also this protruding portion 141 can be regarded as being a tongue that can cause pressure marks on the skin of the body part 5. Moreover, also the misalignment illustrated in Fig. 22 can lead to an improper arrangement of the pressure sensing area 72 on the body part 5. The likelihood of the situations illustrated in Figs. 21 and 22 can be reduced by the tapering of the lateral end portion 76.

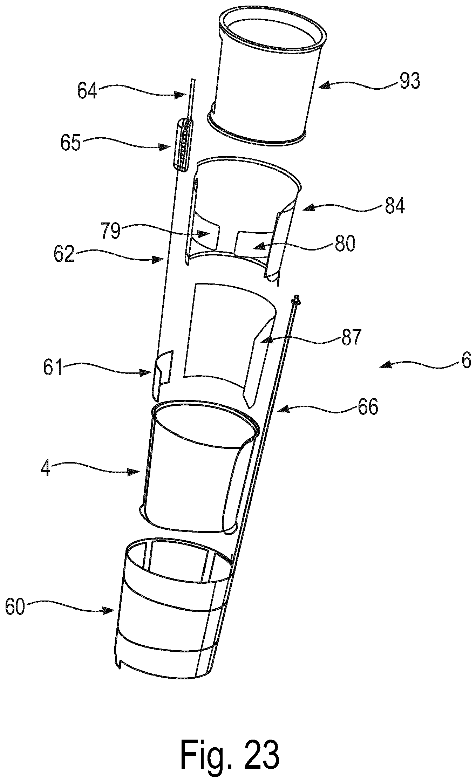

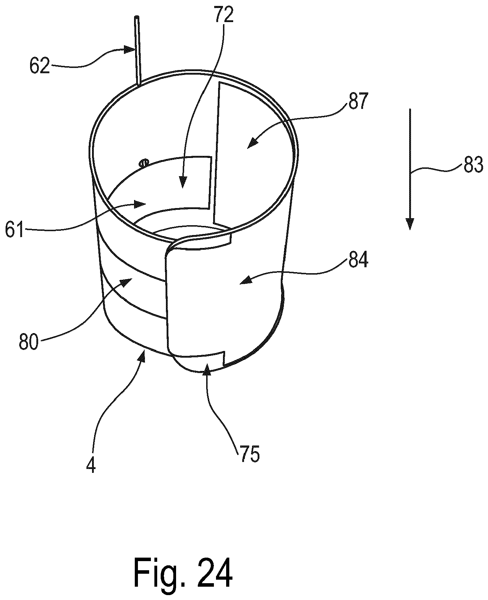

Fig. 23 shows schematically and exemplarily an exploded view of the pressure measuring device 6. As can be seen in Fig. 23, the pressure measuring device 6 also comprises pull tabs 79, 80 which are arranged such that, if a user pulls the pull tabs 79, 80, the diameter of the shell 4 is extended. The pull tab 80 is shown in a larger, partly assembled view of the pressure measuring device 6 in Fig. 24. The pull tab 80 is attached to the lateral end portion 75 of the shell 4 and the other pull tab 79 is attached to the outer surface of the shell 4 in between the overlapping two lateral end portions 75, 76 of the shell 4. The pull tabs 79, 80 are elongate and have a longitudinal axis being perpendicular to the proximal-distal direction 83.

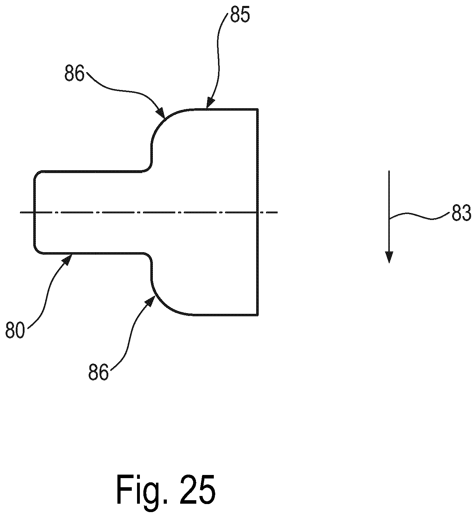

In this embodiment, the pull tabs like the pull tab 80 schematically and exemplarily shown in further detail in Fig. 25 are integrally formed together with attachment elements 85, wherein the attachment elements 85 are attached to the shell 4. The respective attachment element 85 has a rectangular shaped, wherein the respective pull tab, like the pull tab 80 shown in Fig. 25, is adjacent to one side of the rectangular shape being parallel to the proximal-distal direction 83. Preferentially, the corners 86 of the respective attachment element 85 towards the respective pull tab are rounded.

The pull tabs 79, 80 on the shell allow for an improved handling of the pressure measuring device 6 during attachment to the body part 5 of the subject, because by pulling both pull tabs 79, 80 apart, the diameter of the pressure measuring device 6 can be extended more easily. In particular, the medical personnel can more easily slide the pressure measuring device 6 over the subject's hand, align it to the brachial artery and adjust the attachment pressure.

As can be seen in Fig. 24, on the outer surface of the shell 4 a further attachment element 84 like a gluing foil or fabric can be provided, in order to increase the strength of the attachment of the pull tabs to the shell.

As can also be seen in Fig. 24, on the inner side of the shell 4 a sliding foil 87 can be provided which allows for a better sliding of the overlapping lateral end portions 79, 80 relatively to each other. This sliding foil 87 can be a polymer like polytetrafluoroethylene (PTFE). The sliding foil 87 is arranged on the inner surface of the shell 4 between the pressure sensing area 72 and the outer lateral edge of one of the lateral end portions 75, 76. In particular, it is arranged on the inner surface of the lateral end portion 75 being the outer lateral end portion of the two overlapping lateral end portions.

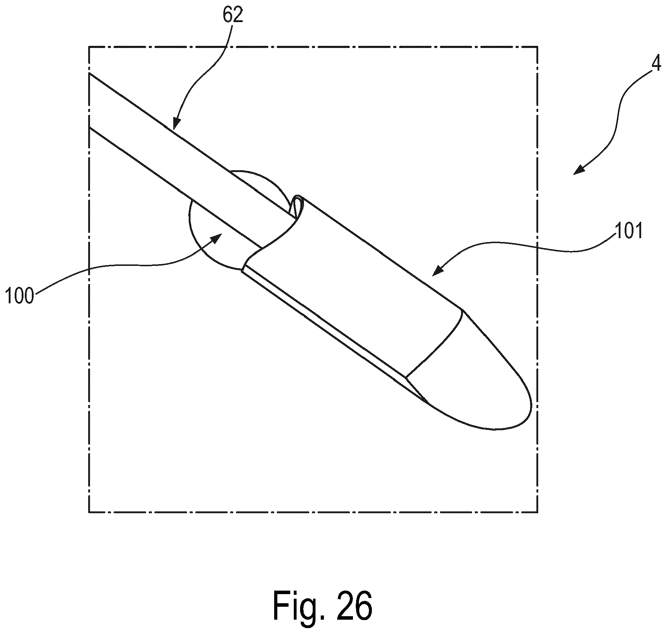

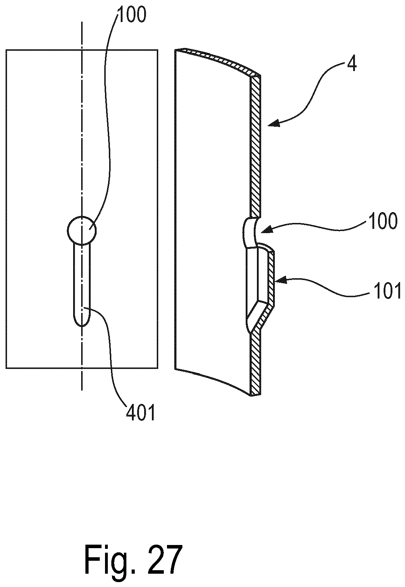

As illustrated in Figs. 26 and 27, in an embodiment the shell 4 comprises an opening 100, 401 partly backed by a tube housing 101 on the outside surface of the shell 4. The opening is formed by a wider first part 100 and a narrower longer opening 401. The first part 100 preferentially is circular and the second part 401 preferentially is longitudinal, i.e., slit-like. The diameter of the first circular part 100 is larger than the width of the slit-like second part 401. The opening 100, 401, i.e., the combination of the first part 100 and the second part 401, therefore can be regarded as being keyhole-like. This is shown especially in the left part of Fig. 27.

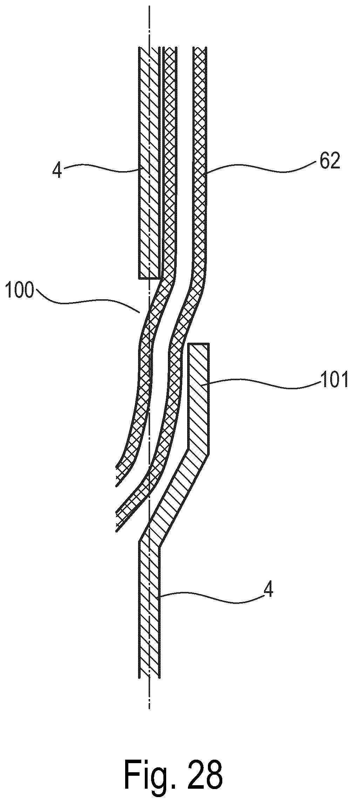

The tube housing 101 is configured to house a part of the tube 62. It covers a section of the opening 100, 401 and preferentially only covers the second part 401 of the opening. Thus, preferentially the tube housing 101 is adjacent to the location of the first part 100 of the opening for housing the part of the tube 62. The tube housing 101 protrudes from the outside of the shell 4. It has a lengthy portion being formed like a part of a hollow cylinder followed by a tapered portion, wherein the entire housing 101 could be regarded as being igloo-like or hangar-like. The housing with the opening can be regarded as being a supportive feed-through bore for the tube 62, wherein this igloo-like or hangar-like construction can prevent a signal loss which generally might be caused by a kink in the tube 62. Moreover, it supports/backs the fluid-filled and hence active area of the pressure sensing pad 61 and thereby also prevents signal loss. Moreover, since the opening together with the housing leads to an embedding of the tube 62 within the shell 4 or the housing of the shell 4 as illustrated in Fig. 28, the likelihood of pressure marks on the body part 5 of the subject can be further reduced. In particular, since the tube 62 is not arranged on the inner surface of the shell 4, pressure marks, which generally might be caused by the tube 62 on the inner surface of the shell 4, can be avoided.

The tube housing 101 can, in addition to the tube 62, also house the layer or foil of the pressure sensing pad directed towards the shell 4, if in an embodiment at the location of the tube housing 101 the tube is already within the pressure sensing pad. This is the case, if the tube 62 has been introduced into the pressure sensing pad such that an end of the tube 62 is within the pressure sensing pad and the tube 62 enters the pressure sensing pad in the region or close to the first part 100 of the opening.

The wider width of the first part 100 of the opening can be used, while manufacturing the pressure measuring device, for feeding the filled curled-up pressure sensing pad through the opening in the shell 4 to enter the inner space surrounded by the curled shell 4.

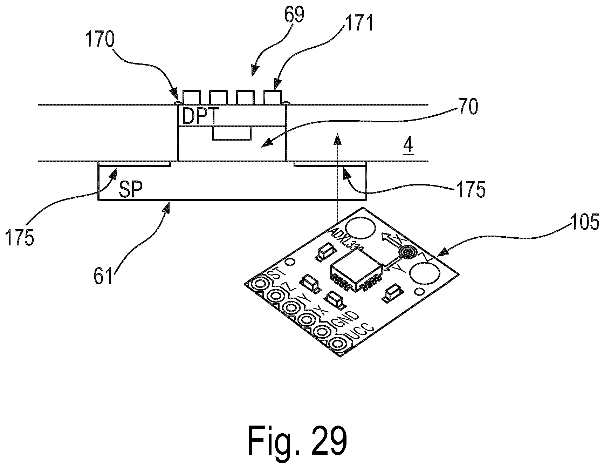

In a further embodiment, which is schematically and exemplarily illustrated in Fig. 29, a pressure transducer 69 is integrated in the shell 4 and located below the pressure sensing pad 61, wherein a fluid 70 like silicone oil is arranged between the pressure sensing pad 61 and the pressure transducer 69 within a cavity in the shell 4 for transmitting pressure from the pressure sensing pad 61 to the pressure transducer 69. This construction can lead to a further increased accuracy of measuring the pressure from the body part 5, because a tube for transmitting the pressure from the pressure sensing pad 61 to a pressure transducer is not required and hence a generally possible kink in the tube, mechanical noise by touching or air bubbles in the tube cannot adversely affect the pressure measurement.

Instead of the fluid 70, also another pressure transmitting material can be used for transmitting pressure from the pressure signal pad to the pressure transducer, wherein the pressure transmitting material can be fluid or non-fluid, especially solid. For instance, the pressure transmitting material can be set, i.e., hardened, silicone oil. The pressure sensing pad 61 can be attached to the shell 4 via an attachment means 175 like, for instance, an adhesive tape. Moreover, the pressure transducer 69 can be fixed in the shell 4 by using glue 170 and connected via connectors 171 to the processor 3.

Thus, a pressure transducer 69 like a differential pressure transducer can be integrated in the shell 4, in order to provide a very short pressure transmission path between the pressure sensing pad 61 and the pressure transducer 69. The fluid 70 between the pressure sensing pad 61 and the pressure transducer 69 allows for an optimal tissue pressure transmission and also protects the sensing membrane of the pressure transducer. The further components of the pressure measuring device 6, which have been described above, can be used together with the integrated pressure transducer 69 shown in Fig. 29. Hence, also if the integrated pressure transducer 69 shown in Fig. 29 is used, the proximal and distal end portions of the shell 4 can be bent away from the body part, the proximal and distal end portions can be softer than a main part of the shell 4 located between the proximal and distal end portions, at least one of the lateral end portions can be tapered, pull tabs can be provided, et cetera.

The pressure measuring device also might comprise a height sensor 105 configured to provide the height of the pressure measuring device, in particular of the shell 4, as schematically and exemplarily illustrated in Fig. 29. The height sensor can include, for instance, an accelerometer and/or a gyrometer. The height sensor 105 can be integrated in the shell 4 of the embodiment illustrated in Fig. 29, but the height sensor can also be integrated in the shell of the embodiments described with reference to the previous figures.

The height sensor values can be used by the processor 3 when determining the physiological parameter based on the measured pressure signal. In particular, the height sensor can be used to continuously track the position of the pressure measuring device relative to the height of the heart, which also may be named "heart level", and then to use this information for determining the physiological parameter like the blood pressure. For instance, the heart level can be measured while the height sensor is arranged at the height of the heart, whereafter differences between the heart level and the height of the height sensor and hence of the sensor pad can be used for correcting the measured blood pressure.

If the processor 3 uses the height as provided by the height sensor, the processor 3 can adjust any height difference to heart level automatically, thereby allowing for a correct determination of the physiological parameter like the blood pressure. In particular, the processor 3 can be configured to, if there is a height difference between the heart level and the height of the pressure sensing pad and hence the of height sensor, calculate a corresponding hydrostatic pressure and subtract the calculated hydrostatic static from or add it to the determined blood pressure value, depending on whether the height of the pressure sensing pad was below or above the heart level, respectively.

The shell 4, which is preferably made from plastic material, such as polyethylene, is a kinking-proof shell preferentially. The thickness of the shell 4 is chosen so that the shell 4 does not buckle when fluid is filled into the fluid bag 88, while at the same time the shell 4 is flexible enough to allow for a certain deformation of the shell 4. That is, when fluid is filled in the fluid bag 88, in order to apply the pressure, the overlapping end portions 75, 76 of the shell 4 move or slide relatively to each other so as to reduce the diameter of the shell 4. However, the shell 4 thereby remains substantially cylindrically shaped, but preferentially slightly tapered or coned towards its distal direction.

Notably, all the above described components of the pressure measuring device 6 are preferably connected to each other in such a way as to form a single unit which can be arranged around the subject's body part 5 for measuring the subject's physiological parameter like, for instance, the blood pressure.

In the following the pressure signal TP, which is measured by using the pressure measuring device 6, and its processing will be exemplarily described in more detail.

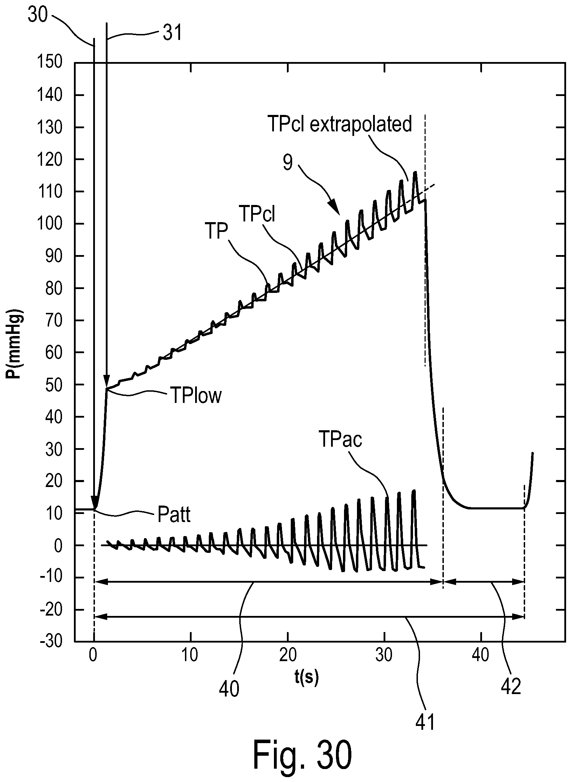

Fig. 30 schematically and exemplarily illustrates the measured pressure P versus time t. Since the pressure is measured on the skin and hence on the tissue, it could also be named tissue pressure TP. The first inflation in the pre-measurement time period starts with a tissue pressure TP being an attachment pressure Patt that is the measured tissue pressure in the uninflated cuff 60. This attachment pressure Patt can range, for instance, from 0 to 15 mmHg. An attachment pressure Patt up to 15 mmHg has turned out to not result in venous congestion for more than 12 hours, thereby making the pressure measuring device 6 optimally suitable for longer term monitoring. For this reason, it is preferred that the attachment pressure Patt is not larger than 15 mmHg. In Fig. 30 the arrow 30 indicates the start of the pre-measurement time period, i.e., the start of the fast inflation period. During this pre-measurement time period the part of the overall tissue pressure range, which has no or substantially no information for the determination of the blood pressure, shall be passed as fast as possible. Therefore, the inflation rate is preferentially as large as possible during the pre-measurement time period. For instance, the inflation rate with respect to the tissue pressure TP can be equal to or larger than 8 mmHg/s. This pre-measurement time period with a fast inflation rate ends at a tissue pressure value which is indicated in Fig. 30 by "TPlow".

The tissue pressure value TPlow also indicates the start of the measurement time period with the slower second inflation rate. In Fig. 30 this start of the measurement time period, which can also be regarded as being a slow inflation period, is indicated by the arrow 31.

In Fig. 30 the time interval 40 indicates the inflation-deflation time period from the start 30 of fast inflation until the tissue pressure TP has dropped below 20 mmHg during the fast deflation, in order to allow venous return. The time period 41 indicates a cycle time being the time between a start of a measurement and a start of a following measurement and the time period 42 indicates a break period being the difference between the inflation-deflation time period 40 and the cycle time 41.

Fig. 30 also illustrates mean pressure TPcl that can be regarded as being a tissue clamping pressure affecting the tissue when the pressure measuring device 6 is attached to the arm 5, for instance, to the upper arm, of the subject. The mean pressure TPcl can be calculated by applying a low pass filter to the tissue pressure TP, wherein the low pass filter might be located within the processor 3. The processor 3 is preferentially configured to determine an alternating component TPac of the tissue pressure by subtracting the mean pressure TPcl from the measured tissue pressure TP, i.e., TPac = TP - TPcl. In Fig. 30 the TPac curve is shown enlarged by a factor of 2, in order to improve visibility of this curve.

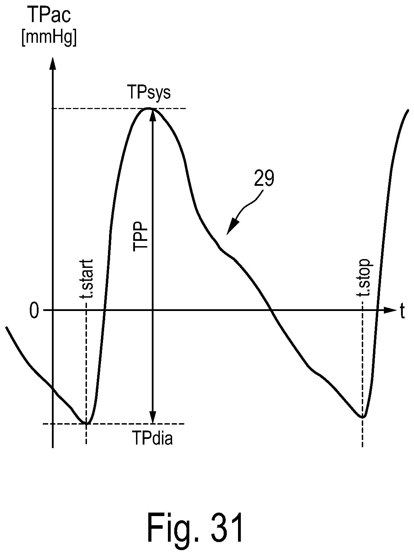

During the slow inflation period, i.e., during the measurement time period, TP pulse curves and/or TPac pulse curves are recorded, which may be analyzed simultaneously, i.e., online, wherein the TP pulse curves and/or the TPac pulse curves have tissue pressure waveforms (TPW) comprising information which allows for an accurate determination of the respiration rate. Since the blood pressure is noninvasively measured (although it nevertheless has an accuracy which is comparable to the accuracy of invasively measured blood pressure), it can be abbreviated with niBP meaning "noninvasive blood pressure". A TPac pulse curve is schematically and exemplarily shown in Figs. 31 to 34.

In the following the TPac pulse curves are used for determining features for the pressure pulses 9. The TPac pulse curves can therefore be regarded as being feature determination pulse curves or feature determination pulses. Figs. 31 to 34 hence show feature determination pulse curves or feature determination pulses 29 being TPac pulses. In another embodiment the feature determination pulses can also directly be the TP pulse curves, i.e., the pressure pulses 9 can be directly used for determining the features. The processor 3 is configured to determine for a respective pressure pulse of the plurality of pressure pulses 9 at least one feature which characterizes the respective pressure pulse.

In an embodiment, the processor 3 is configured to determine a difference TPP between a maximum measured pressure and a minimum measured pressure of the respective feature determination pulse 29 as a feature for the respective pressure pulse. This feature will be described with reference to Fig. 31 in the following.

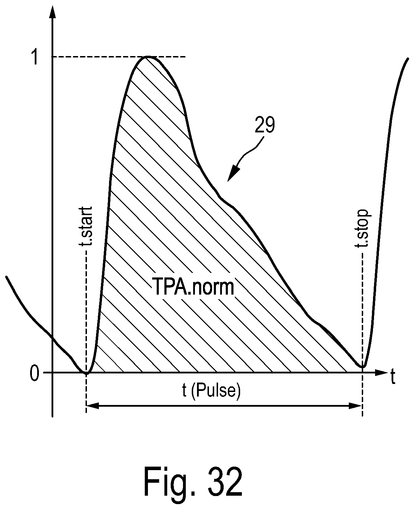

The difference TPP is a difference of a feature determination pulse's maximum systolic pressure (TPsys) and the feature determination pulse's pressure at an end-diastolic point (TPdia), which is preferably the minimum pressure of the respective feature determination pulse. In Fig. 31 and also in Figs. 32 to 34 the terms "t.start" and "t.stop" indicate the start and the end, respectively, of the respective pulse 29.

The processor 3 can also be configured to determine the pulse duration (t(Pulse)) being the time difference between an end-diastolic point and a following end-diastolic point of the feature determination pulse 29. This feature can also be defined as being the temporal difference between the start of the respective pulse (t.start) and the end of the respective pulse (t.stop). This feature is indicated in Fig. 32.

The processor 3 can also be adapted to determine the pulse area (TPA) of the respective pulse 29 being the area under the respective pulse curve within the time defined by t.start to t.stop and ranging from the feature determination pulse's pressure at an end-diastolic point (TPdia) to a feature determination pulse's maximum systolic pressure (TPsys). Preferentially, the pulse area TPA is scaled to TPP=1 as indicated in Fig. 32. This scaled pressure pulse area is named "TPA.norm".

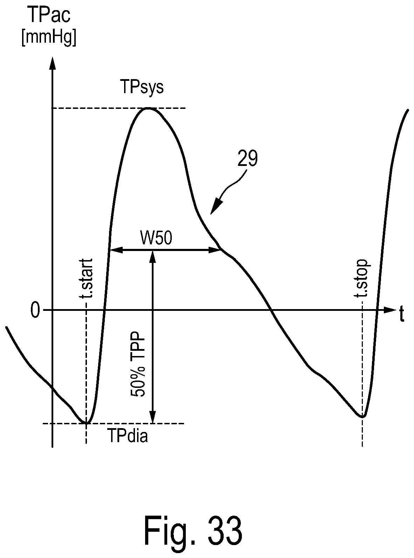

The processor 3 can also be adapted to determine the pulse width at half maximum (W50) of the respective pulse 29 as indicated in Fig. 33.

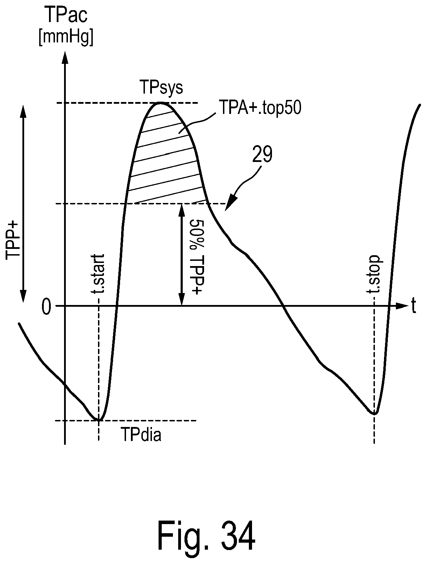

Moreover, the processor 3 can be adapted to determine the area TPA+.top50 enclosed by an upper part of the feature determination pulse 29, as schematically illustrated in Fig. 34. The upper end of the area TPA+.top50 enclosed by the upper part is at the feature determination pulse's 29 maximum systolic pressure TPsys and the lower end of the area TPA+.top50 of the upper part is between the feature determination pulse's 29 maximum systolic pressure TPsys and a pressure value which corresponds to the mean TPcl of the measured pressure TP. Since the feature determination pulse 29 has been determined by subtracting the mean TPcl from the measured pressure TP, the pressure value, which corresponds to the mean TPcl of the measured pressure TP, is zero. Preferentially, the processor 3 is configured to determine the area TPA+.top50 enclosed by the upper part of the feature determination pulse such that the lower end of the upper part is at a pressure value which corresponds to half of the pressure distance TPP+ between the feature determination pulse's maximum systolic pressure TPsys and the pressure value which corresponds to the mean TPcl of the measured pressure TP.

Figs. 31 to 34 show a TPac pulse curve such that in this example also the features have been defined based on the TPac pulse curve. However, as mentioned above, it is also possible to define these or other features based on a TP pulse curve.

The processor 3 is further configured to determine for a respective pressure pulse a blood pressure determination value TPWP_M based on at least one determined feature such that for several pressure pulses, which are present at different times, several blood pressure determination values TPWP_M are determined, wherein the several blood pressure determination values TPWP_M determined for the several pressure pulses and hence for several times form a blood pressure determination curve TPW_M-curve. The processor 3 is configured to determine the blood pressure based on the blood pressure determination curve TPW_M-curve, for instance as explained in WO 2018/210931 A1. In particular, the processor 3 is configured to determine a position of a maximum (TPW_M-curve.max) of the blood pressure determination curve TPW_M-curve and to determine the blood pressure based on the determined position and the measured pressure TP. For instance, the processor 3 is configured to determine the systolic arterial blood pressure (SAPni) based on the mean TPcl of the measured pressure TP at the determined position of the maximum (TPW_M-curve.max) in accordance with following equation:

$$\text{SAPni} = \alpha \cdot (\text{TPcl@TPW\_M-curve.max}) \quad , \quad (1)$$

wherein the parameter $\alpha$ can be predetermined by calibration.

Preferentially the processor is configured to provide a function which receives as input the at least one feature of a respective pressure pulse and which outputs a respective blood pressure determination value which forms, together with the blood pressure determination values determined for the other pressure pulses, the blood pressure determination curve. The function has at least one parameter which can be determined by calibration, wherein by invasive means reference blood pressure values are determined very accurately and the at least one parameter is determined such that the apparatus yields the very accurately invasively measured blood pressure values with high statistical precision and accuracy.

In a preferred embodiment the processor 3 is configured to form the blood pressure determination curve TPW_M-curve based on blood pressure determination values TPWP_M which depend only on the feature being the difference TPP between the feature determination pulse's maximum systolic pressure TPsys and the feature determination pulse's pressure at the end-diastolic point TPdia. Thus, in an embodiment the function, which is used for determining the blood pressure determination values TPWP_M, depends on TPP, but not on any other features of the pressure pulses. In particular, the blood pressure determination values TPWP_M can be determined in accordance with following equation:

$$\text{TPWP\_M} = d1 \cdot \text{TPP}^{\text{exp1}}, \quad (2)$$

wherein d1 and exp1 are predetermined constants. For instance, d1 and exp1 can be predetermined by calibration. However, it has been found that they can also both be chosen to be equal to one, i.e., d1 =1=exp1, such that TPWP_M = TPP.

Moreover, in a preferred embodiment the processor 3 is configured to form the blood pressure determination curve TPW_M-curve based on blood pressure determination values TPWP_M which depend only on a) the feature being the difference TPP between the feature determination pulse's maximum systolic pressure TPsys and the feature determination pulse's pressure at the end-diastolic point TPdia and b) the feature being the area TPA+.top50 enclosed by the upper part of the feature determination pulse. Thus, in an embodiment the function, which is used for determining the blood pressure determination values TPWP_M, depends on TPP and TPA+.top50, but not on further features of the pressure pulses. In particular, the blood pressure determination values TPWP_M can be determined in accordance with following equation:

$$TPWP\_M = d2 \cdot TPA+.top50^{exp2} \cdot TPP^{exp3}, \quad (3)$$

wherein d2, exp2 and exp3 are predetermined constants. For instance, d2 can be equal to one, i.e., d2 = 1, wherein exp2 and exp3 can be predetermined by calibration. However, it is also possible that the constant d2 is determined by calibration. In a preferred embodiment, d2 and exp3 are both equal to one (d2=1, exp3=1) and exp2 is equal to 0.7 (exp2=0.7).

In an embodiment the blood pressure determination curve TPW_M-curve can be a smoothed curve such that the maximum of the blood pressure determination curve is also smoothed. The smoothing procedure used for smoothing the blood pressure determination curve can include, for instance, filtering and/or fitting. Further, several pressure pulses can be considered, after the smoothed maximum, to detect the smoothed maximum unambiguously wherein the number of these several pulses can be predetermined or can depend on the heart rate of the subject. For instance, this number can increase with increasing heart rate. In an embodiment this number is 3 for a heart rate of 60/min and 10 for a heart rate of 200/min.

Preferentially, the blood pressure measurement is intended to be used in a sequence of measurements in rapid succession to allow for effective semi-continuous blood pressure monitoring such that the stress for the monitored individual, i.e., for the monitored subject, is minimized.

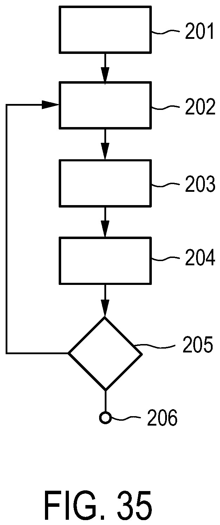

In the following an embodiment of a method for determining a physiological parameter of a subject will be exemplary described with reference to a flowchart shown in Fig. 35.

In step 201, the pressure measuring device 6 is arranged around the body part 5 of the subject. In step 202, a pressure signal of the subject, i.e., the tissue pressure TP, is measured by using the pressure measuring device 6, wherein the measured pressure signal is indicative of blood pulsations. In particular, the pressure applicator element 60, especially the fluid bag 88, applies increasing or decreasing pressure to the body part 5, while the tissue pressure TP is measured at the pressure sensing area 72 of the shell 4. In step 203, the physiological parameter, for instance the blood pressure, is determined based on the measured pressure signal by the processor 3 of the apparatus 1 for determining a physiological parameter of the subject. In step 204, the determined physiological parameter can be output to the medical personnel via an output unit like the display 22.

In step 205, it is determined whether an abort criterion is fulfilled. If this is the case, the method stops in step 206, otherwise it proceeds with step 202. Thus, the method can be carried out in a loop for continuously measuring the tissue pressure and for continuously monitoring the physiological parameter like the blood pressure over time in several measurement cycles, until the abort criterion is fulfilled. For instance, the measurement and monitoring can be interrupted, if a user like a physician has input a corresponding command into the apparatus via an input unit like a keyboard, a computer mouse, a touchpad, et cetera.

Since steps 201, 202, 205 and 206 provide the measured pressure signal until the abort criterion is fulfilled, these steps could also be regarded as being steps of a pressure measuring method for measuring pressure of a subject.

The pressure measuring device allows for a hydraulic coupling of the pressure measurement, wherein a transcutaneous high-fidelity recording of the arterial pressure waveform can be possible, in which even the closure of the aortic valve, i.e., the dicrotic notch, can be visible.

Although in above described embodiments the determined physiological parameter is the blood pressure, also another physiological parameter can be determined like the respiration rate, the heart rate, the fluid responsiveness, the cardiac output and contraction force, the arterial resistance, et cetera. For more details regarding the measurement of the different physiological parameters based on the measured tissue pressure reference is made to, for instance, WO 2018/210931 A1, WO 2019/211210 A1, WO 2021/255064 A1 and WO 2022/117471 A1, which are herewith incorporated by reference.

Although in above described embodiments features are determined by using the TPac pulses as processed pressure pulses, the features can also be determined by using directly the measured pressure pulses, i.e., for instance, the TP pulses. It is also possible to process the pressure pulses in another way, i.e., for example, not by subtracting the mean TP value for determining the TPac pulses. For instance, for the respective measured pressure pulse the pressure values at t.start and t.stop can be connected by a straight line and this straight line can be subtracted from the respective measured pressure pulse, in order to determine a processed pressure pulse.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of features of pulses, of certain curves, of the physiological parameter, et cetera performed by one or several units or devices can be performed by any other number of units or devices. The calculations and determinations and/or the control of the apparatus for determining the physiological parameter of the subject in accordance with the method for determining the physiological parameter of the subject and/or the control of the pressure measuring device in accordance with the pressure measuring method can be implemented as program code means of a computer and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a pressure measuring device configured to surround a subject's body part. It comprises a pressure application element configured to apply pressure to the body part, a pressure sensing device configured to measure a pressure signal of the body part and a shell which is arranged to be located between the pressure application element and the body part. The inner surface of the shell comprises a pressure sensing area in which at least a part of the pressure sensing device is located. Preferentially, the pressure sensing area is closer to the distal edge of the shell than to the proximal edge of the shell. Placing the pressure sensing area in this way can allow for an improved pressure signal.

**Claims**

1. A pressure measuring device configured to surround a subject's body part, wherein the pressure measuring device comprises:

   - a pressure application element (60) configured to apply pressure to the body part (5),
   - a pressure sensing device configured to measure a pressure signal of the subject, wherein the measured pressure signal is indicative of blood pulsations,
   - a shell (4) which is arranged to be located between the pressure application element (60) and the body part (5), when the pressure measuring device (6) surrounds the body part (5), wherein the shell (4) is curled to a cylindrical or conical configuration and wherein the inner surface of the shell (4) comprises a pressure sensing area (72) in which at least a part (61) of the pressure sensing device is located.

2. The pressure measuring device as defined by any of the preceding claims, wherein the shell (4) comprises a distal edge (124) and a proximal edge (125) and wherein the pressure sensing area (72) is closer to the distal edge (124) of the shell (4) than to the proximal edge (125) of the shell (4).

3. The pressure measuring device as defined by any of claims 1 and 2, wherein the shell (4) comprises a distal edge (124) and a proximal edge (125), wherein also the pressure sensing area (72) comprises a distal edge (123) and a proximal edge, wherein the distance between the distal edge (123) of the pressure sensing area (72) and the distal edge (124) of the shell (4) is within a range from 15% to 20% of the entire height of the shell (4) from its distal edge (124) to its proximal edge (125).

4. The pressure measuring device as defined by any of the preceding claims, wherein the pressure sensing area has a length between 20% and 50% of the inner circumference of the shell in the circumferential direction and/or a length between 15% to 40% of the entire shell height in the proximal-distal direction.

5. The pressure measuring device as defined by any of the preceding claims, wherein the pressure sensing device comprise a fluid-filled pressure sensing pad (61) arranged in the pressure sensing area (72) on the inner surface of the shell (4).

6. The pressure measuring device as defined by claim 5, wherein the pressure sensing pad (61) is filled with fluid having a fluid thickness, in a flat condition of the pressure sensing pad (61), within a range from 0.90 mm to 1.10 mm.

7. The pressure measuring device as defined by any of claims 5 and 6, wherein a mesh (68) is arranged within the pressure sensing pad (61).

8.  The pressure measuring device as defined by claim 7, wherein the mesh (68) is a three-dimensional mesh.

9.  The pressure measuring device as defined by any of claims 5 to 8, wherein the pressure sensing device comprises a pressure transducer located in a pressure transducer housing (63) and a fluid-filled tube (62) for guiding pressure from the pressure sensing pad (61) to the pressure transducer, wherein a) the pressure sensing pad (61) and the tube (62) and b) the pressure transducer housing (63) are filled with different fluids having different viscosities.

10. The pressure measuring device as defined by claim 9, wherein the fluid in the pressure sensing pad (61) and the tube (62) has a lower viscosity than the fluid in the pressure transducer housing (63).

11. The pressure measuring device as defined by any of the preceding claims, wherein the pressure measuring device (6) comprises a first mark (121) having an angular offset within a range from 35° to 45° relative to the location of the center (98) of the pressure sensing area (72).

12. The pressure measuring device as defined by claim 11, wherein the pressure measuring device (6) comprises a second mark (122) having an angular offset within a range from 35° to 45° relative to the location of the center (98) of the pressure sensing area (72), wherein the angular offset of the first and second marks (121, 122) is in opposite angular directions.

13. An apparatus for determining a physiological parameter of a subject, wherein the apparatus comprises:

    - a pressure measuring device (6) as defined by any of claims 1 to 12 for measuring a pressure signal of the subject over a time, wherein the measured pressure signal is indicative of blood pulsations,
    - a processor (3) configured to determine the physiological parameter based on the measured pressure signal.

14. A pressure measuring method for measuring pressure of a subject, wherein the method comprises:

    - arranging a pressure measuring device (6) as defined in any of claims 1 to 12 around a body part (5) of the subject,
    - applying pressure to the body part (5) by a pressure application element (60) of the pressurization device of the pressure measuring device (6),
    - measuring a pressure signal of the subject, wherein the measured pressure signal is indicative of blood pulsations, by the pressure sensing device of the pressure measuring device (6).

15. A method for determining a physiological parameter of a subject, wherein the method comprises:

    - applying the pressure measuring method as defined by claim 14, thereby measuring a pressure signal of the subject over a time, wherein the measured pressure signal is indicative of blood pulsations,
    - determining the physiological parameter based on the measured pressure signal by a processor (3).

Fig. 1

Fig. 2

Fig. 3

Fig. 4

193

93

4

Fig. 5

Fig. 6

Fig. 7

Fig. 8

Fig. 9

Fig. 10

Fig. 11

Fig. 12

Fig. 13

Fig. 14

73

76

SEE DETAIL A

4

77

SEE DETAIL B

74

Fig. 15

73

formed edge
width

formed edge
height

DETAIL A

# Fig. 16

formed edge
width

74

formed edge
height

DETAIL B

# Fig. 17

Fig. 18

Fig. 19

Fig. 20

Fig. 21

Fig. 22

Fig. 23

Fig. 24

85

86

80

86

83

Fig. 25

Fig. 26

Fig. 27

Fig. 28

Fig. 29

Fig. 30

Fig. 31

Fig. 32

Fig. 33

Fig. 34

FIG. 35

Europäisches Patentamt

European Patent Office

Office européen des brevets

# EUROPEAN SEARCH REPORT

**Application Number**

EP 24 18 1791

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/218182 A1 (PFEIFFER ULRICH JOACHIM [NL] ET AL) 13 July 2023 (2023-07-13) * abstract * * figures 1,2,4,10 * * paragraph [0058] * * paragraph [0084] * * paragraph [0088] * | 1-15 | INV. A61B5/022 A61B5/023 |
| X,D | WO 2014/121945 A1 (UP MED GMBH [DE]) 14 August 2014 (2014-08-14) * abstract * * figures 2,3,9 * | 1-15 | |
| X | WO 2024/008607 A1 (KONINKLIJKE PHILIPS NV [NL]) 11 January 2024 (2024-01-11) * abstract * * figures 18,22 * | 1-15 | |
| X | US 2019/090762 A1 (SAWANOI YUKIYA [JP] ET AL) 28 March 2019 (2019-03-28) * abstract * * figures 2,12 * * paragraph [0035] - paragraph [0037] * * paragraph [0043] * * paragraph [0050] - paragraph [0051] * | 1-13,15 | TECHNICAL FIELDS SEARCHED (IPC) A61B |
| A | US 2010/106029 A1 (FRADEN JACOB [US]) 29 April 2010 (2010-04-29) * abstract * * figure 4 * | 1-4, 13-15 | |
| A | US 8 998 817 B2 (PFEIFFER ULRICH [DE]; THOMAMUELLER TOBIAS [DE] ET AL.) 7 April 2015 (2015-04-07) * abstract * * figure 4 * | 1-4, 13-15 | |

-/--

The present search report has been drawn up for all claims

| Place of search | Date of completion of the search | Examiner |
|---|---|---|
| The Hague | 26 November 2024 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | **Application Number** EP 24 18 1791 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/222450 A1 (KONINKLIJKE PHILIPS NV [NL]) 23 November 2023 (2023-11-23)<br>* abstract; figure 5 *<br>* page 5, line 37 *<br>* page 7, line 20 - line 28 *<br>----- | 7-10 | |
| A | EP 4 278 961 A1 (KONINKLIJKE PHILIPS NV [NL]) 22 November 2023 (2023-11-22)<br>* abstract; figure 5 *<br>* paragraph [0032] - paragraph [0033] *<br>* paragraph [0039] *<br>----- | 7-10 | |
| A | KR 2019 0072002 A (NAM HWA SEON [KR]) 25 June 2019 (2019-06-25)<br>* abstract *<br>* paragraph [0007] *<br>----- | 7,8 | |

TECHNICAL FIELDS SEARCHED (IPC)

The present search report has been drawn up for all claims

| Place of search | Date of completion of the search | Examiner |
|---|---|---|
| The Hague | 26 November 2024 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 1791

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2024

| Patent document cited in search report | | | Publication date | Patent family member(s) | | | Publication date |
|---|---|---|---|---|---|---|---|
| US | 2023218182 | A1 | 13-07-2023 | CN | 115701937 | A | 14-02-2023 |
| | | | | EP | 3925527 | A1 | 22-12-2021 |
| | | | | EP | 4167841 | A1 | 26-04-2023 |
| | | | | JP | 2023529974 | A | 12-07-2023 |
| | | | | US | 2023218182 | A1 | 13-07-2023 |
| | | | | WO | 2021255064 | A1 | 23-12-2021 |
| WO | 2014121945 | A1 | 14-08-2014 | CN | 105228516 | A | 06-01-2016 |
| | | | | CN | 110811586 | A | 21-02-2020 |
| | | | | JP | 6360838 | B2 | 18-07-2018 |
| | | | | JP | 2016509516 | A | 31-03-2016 |
| | | | | US | 2015359446 | A1 | 17-12-2015 |
| | | | | US | 2022257130 | A1 | 18-08-2022 |
| | | | | WO | 2014121805 | A1 | 14-08-2014 |
| | | | | WO | 2014121945 | A1 | 14-08-2014 |
| WO | 2024008607 | A1 | 11-01-2024 | NONE | | | |
| US | 2019090762 | A1 | 28-03-2019 | CN | 109195514 | A | 11-01-2019 |
| | | | | DE | 112017002688 | T5 | 07-03-2019 |
| | | | | JP | 6610433 | B2 | 27-11-2019 |
| | | | | JP | 2017209434 | A | 30-11-2017 |
| | | | | US | 2019090762 | A1 | 28-03-2019 |
| | | | | WO | 2017203958 | A1 | 30-11-2017 |
| US | 2010106029 | A1 | 29-04-2010 | CN | 101730502 | A | 09-06-2010 |
| | | | | EP | 2139387 | A1 | 06-01-2010 |
| | | | | JP | 2010522610 | A | 08-07-2010 |
| | | | | KR | 20100027093 | A | 10-03-2010 |
| | | | | TW | 200843697 | A | 16-11-2008 |
| | | | | US | 2010106029 | A1 | 29-04-2010 |
| | | | | WO | 2008121454 | A1 | 09-10-2008 |
| US | 8998817 | B2 | 07-04-2015 | NONE | | | |
| WO | 2023222450 | A1 | 23-11-2023 | NONE | | | |
| EP | 4278961 | A1 | 22-11-2023 | NONE | | | |
| KR | 20190072002 | A | 25-06-2019 | NONE | | | |

EPO FORM P0459

For more details about this annex : see Official Journal of the European Patent Office, No. 12/82

**REFERENCES CITED IN THE DESCRIPTION**

**Patent documents cited in the description**

- WO 2014121945 A1
- WO 2018210931 A1
- WO 2019211210 A1
- WO 2021255064 A1
- WO 2022117471 A1